(19) Europäisches Patentamt
European Patent Office
Office européen des brevets

(11) Veröffentlichungsnummer: **0 003 002**
**B1**

(12) # EUROPÄISCHE PATENTSCHRIFT

(45) Veröffentlichungstag der Patentschrift: **13.06.84**

(21) Anmeldenummer: **78810031.1**

(22) Anmeldetag: **18.12.78**

(51) Int. Cl.³: **C 08 F 2/50,** C 07 C 49/82, C 07 C 69/00, C 07 C 97/00, C 07 C 103/42, C 07 C 121/00, C 07 C 143/78, C 07 C 147/103, C 07 C 149/20, C 07 D 307/82, C 07 D 311/22

(54) **Verwendung von aromatisch-aliphatischen Ketonen als Photoinitiatoren, photopolymerisierbare Systeme enthaltend solche Ketone und neue aromatisch-aliphatische Ketone.**

(30) Priorität: **22.12.77 CH 15884/77**
**08.03.78 CH 2518/78**

(43) Veröffentlichungstag der Anmeldung:
**11.07.79 Patentblatt 79/14**

(45) Bekanntmachung des Hinweises auf die Patenterteilung
**13.06.84 Patentblatt 84/24**

(84) Benannte Vertragsstaaten:
**BE CH DE FR GB IT NL SE**

(56) Entgegenhaltungen:
**CH - A - 459 750**
**FR - A - 2 013 828**
**FR - A - 2 022 723**
**FR - A - 2 028 477**
**FR - A - 2 156 760**
**FR - A - 2 162 609**
**FR - A - 2 209 789**
**US - A - 3 657 088**

(73) Patentinhaber: **CIBA-GEIGY AG**
**Postfach**
**CH-4002 Basel (CH)**

(72) Erfinder: **Felder, Louis, Dr.**
**Riehenring 5**
**CH-4058 Basel (CH)**
Erfinder: **Kirchmayr, Rudolf, Dr.**
**Ettingerstrasse 9**
**CH-4147 Aesch (CH)**
Erfinder: **Hüsler, Rinaldo, Dr.**
**Belchenstrasse 12**
**CH-4054 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 003 002 B1

## Beschreibung

Die Erfindung betrifft die Verwendung aromatischaliphatischer Ketone, die in der $\alpha$-Stellung substituiert sind, als Initiatoren für die Photopolymerisation ungesättigter Verbindungen oder die photochemische Vernetzung von Polyolefinen sowie die solche Initiatoren enthaltenden photopolymerisierbaren bzw. vernetzbaren Systeme.

Photochemische Polymerisationsprozesse haben in der Technik erhebliche Bedeutung erlangt, vor allem in solchen Fällen, wo dünne Schichten in kurzer Zeit gehärtet werden müssen, wie z.B. bei der Härtung von Lacküberzügen oder bei Trocknung von Druckfarben. Die UV-Bestrahlung in Gegenwart von Photoinitiatoren zeigt gegenüber herkömmlichen Härtungsverfahren eine Reihe von Vorteilen, deren wichtigster wohl die hohe Geschwindigkeit der Photohärtung ist. Die Geschwindigkeit ist stark vom verwendeten Photoinitiator abhängig und es hat nicht an Versuchen gefehlt, die herkömmlichen Initiatoren durch immer bessere und wirksamere Verbindungen zu ersetzen. Zu den wirksamsten Photoinitiatoren gehören Derivate des Benzoins, vor allem Benzoin-äther wie sie beispielsweise in der DT—PS 1 694 149 beschrieben werden, Derivate des $\alpha$-Hydroxymethylbenzoins wie die in der DT—OS 1 923 266 beschrieben sind, sowie Dialkoxyacetophenone und Benzil-monoketale, wie sie beispielsweise in der DT—OS 2 261 383 oder 2 232 365 beschrieben sind. $\alpha$-Aminoacetophenone und $\alpha$-Diaminoacetophenone wurden kürzlich im US Patent 4 048 034 und $\alpha$-Hydroxy-$\alpha$-alkylolacetophenone und deren Aether in der DT—OS 2 357 866 als Photoinitiatoren vorgeschlagen.

Nachteile, die diesen bekannten Photoinitiatoren anhaften, sind z.T. ungenügende Dunkellagerstabilität der mit solchen Initiatoren vermischten photopolymerisierbaren Systeme. Einige Benzoinderivate neigen zur Vergilbung der gehärteten Massen. Andere Initiatoren besitzen unzureichende Reaktivität, was sich in relativ langen Härtungszeiten äussert, oder sie sind in den photopolymerisierbaren Systemen zu wenig löslich oder sie werden durch Luftsauerstoff rasch inaktiviert.

In der Technik besteht daher ein Bedarf an Photoinitiatoren, die im Substrat gut löslich sind und bei guter Dunkellagerstabilität die Photopolymerisation rascher auslösen und eine höhere Polymerausbeute pro Zeiteinheit ergeben als die bekannten Photoinitiatoren. Durch die Verwendung solcher verbesserter Photoinitiatoren würden sich die kostspieligen industriellen UV-Bestrahlungsanlagen besser ausnützen lassen.

Es wurde gefunden, dass Verbindungen der nachstehenden Formeln I, II, III und IV die geforderten Eigenschaften als Photoinitiatoren besitzen, vor allem eine schnelle Polymerisation bewirken und die geschilderten Nachteile nicht oder in wesentlich geringerem Masse aufweisen als die bekannten Photoinitiatoren. Sie eignen sich ausserdem zur photochemischen Vernetzung von Polyolefinen. Die Erfindung betrifft die Verwendung von Berbindungen der Formeln I, II, III oder IV

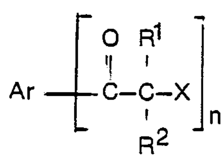
I

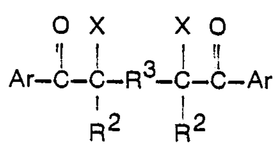
II

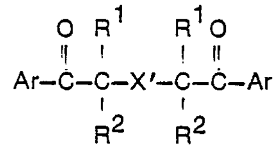
III

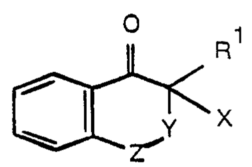
IV

worin n 1 oder 2 ist,

Ar, wenn n = 1 ist, Aryl mit 6—14 C-Atomen, durch Cl, Br, CN, OH, Alk, —OAlk, —SAlk, —$SO_2$Alk, —$SO_2$Phenyl, —COOAlk, —$SO_2NH_2$, —$SO_2$NHAlk, —$SO_2$N$(Alk)_2$, —NHAlk, —N$(Alk)_2$, Phenoxy oder —NHCOAlk substituiertes Phenyl oder Thienyl, Pyridyl oder Furyl darstellt und wenn n = 2 ist, Arylen mit 6—12 C-Atomen oder eine Gruppe -Phenylen-T-Phenylen- bedeutet,

Alk einen Alkylrest mit 1—4 C-Atomen bedeutet,

X eine der Gruppen —$NR^4R^5$, —$OR^6$, —O—Si$(R^7)(R^8)_2$, Hydroxymethoxy, ($C_1$—$C_4$-Alkoxy)-methoxy, ($C_2$—$C_8$-Acyloxy)-methoxy oder zusammen mit $R^1$ —OCH($C_1$—$C_8$-Alkyl)—O$(CH_2)_{1-2}$—, —OCH($C_6C_{14}$-Aryl)—O—$(CH_2)_{1-2}$—, —OCH($C_1$—$C_8$-Alkyl)— oder —OCH($_6C_{14}$—Aryl)— darstellt,

X' eine der Gruppen —$NR^{10}$—, —N(Phenyl)—,

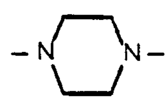

oder —N$(R^{10})$—$R^{11}$—N$(R^{10})$— darstellt,

Y eine direkte Bindung oder —$CH_2$— darstellt,

Z —O—, —S—, —$SO_2$—, —$CH_2$— oder —C$(CH_3)_2$— darstellt,

T —O—, —S—, —$SO_2$—, —$CH_2$— oder —CH=CH— darstellt,

$R^1$ in Formel I bei n gleich 1 und X gleich —$OR^6$ $C_1$—$C_8$ alkyl, das durch $C_2$—$C_8$ Acyloxy,

—COO—($C_1$—$C_4$)Alkyl oder —CN substituiert sein kann, $C_5$—$C_6$ Cycloalkyl oder $C_7$—$C_9$ Phenylalkyl bedeutet, und in allen anderen Fällen Alkyl mit 1—8 C-Atomen, das durch OH, $C_1$—$C_4$ Alkoxy, $C_2$—$C_8$ Acyloxy, —COO—($C_1$—$C_4$)Alkyl oder —CN substituiert sein kann, Cycloalkyl mit 5—6 C-Atomen oder Phenylalkyl mit 7—9 C-Atomen bedeutet,

$R^2$ eine der für $R^1$ gegebenen Bedeutungen hat oder Allyl, Methallyl, 2-Carbamoyläthyl, 2-(N—$C_1$—$C_4$-Alkylcarbamoyl)-äthyl, 2-(N,N—Di—$C_1$—$C_4$-Alkylcarbamoyl)-äthyl, 2-(2-Pyridyl)-äthyl, 2-(2-Oxo-1-pyrrolidinyl)-äthyl oder 2-(Di—O—$C_1$—$C_4$-alkylphosphono)-äthyl ist, oder $R^1$ und $R_2$ zusammen Alkylen mit 4—6 C-Atomen oder Oxa- oder Azaalkylen mit 3—4 C-Atomen bedeutet, oder $R^1$ und $R^2$ in Formel I beide Hydroxymethyl sind,

$R^3$ eine direkte Bindung, Alkylen mit 1—6 C-Atomen, $C_2$—$C_6$-Oxaalkylen, $C_2$—$C_6$-Thiaalkylen, $C_2$—$C_6$—S-Oxothiaalkylen, $C_2$—$C_6$—S,S-Dioxo-thiaalkylen oder Cyclohexylen bedeutet, oder zusammen mit den beiden Substituenten $R^2$ und den beiden C-Atomen, an die diese Substituenten gebunden sind, einen Cyclopentan- oder Cyclohexanring bildet,

$R^4$ Alkyl mit 1—12 C-Atomen, durch —OH oder —OAlk substituiertes Alkyl mit 2—4 C-Atomen, Allyl, Cyclohexyl, Phenylalkyl mit 7—9 C-Atomen, Phenyl oder durch Cl, Alk, OH, —OAlk oder —COOAlk substituiertes Phenyl bedeutet,

$R^5$ Alkyl mit 1—12 C-Atomen, durch —OH oder —OAlk substituiertes Alkyl mit 2—4 C-Atomen, Allyl, Cyclohexyl oder Phenylalkyl mit 7—9 C-Atomen bedeutet, oder zusammen mit $R^4$ Alkylen mit 4—5 C-Atomen bedeutet, das durch —O—, —NH— oder —NAlk— underbrochen sein kann, oder im Falle von Verbindungen der Formel I zusammen mit $R^2$ Alkylen oder Phenylalkylen mit 1—9 C-Atomen oder Oxa- oder Azaalkylen mit 2—3 C-Atomen bedeutet,

$R^6$ Wasserstoff, Alkyl mit 1—12 C-Atomen, durch —OH oder —OAlk substituiertes Alkyl mit 2—4 C-Atomen, Allyl, Cyclohexyl, Phenylalkyl mit 7—9 C-Atomen, Phenyl oder durch Cl oder Alk substituiertes Phenyl bedeutet,

$R^7$ und $R^8$ gleich oder verschieden sind und Alkyl mit 1—4 C-Atomen oder Phenyl bedeuten,

$R^{10}$ Alkyl mit 1—8 C-Atomen, Cyclohexyl oder Benzyl und

$R^{11}$ Alkylen mit 2—8 C-Atomen, Xylylen, Phenylen oder eine Gruppe -Phenylen-T-Phenylen- darstellt, mit Ausnahme der Verbindungen der Formel I, worin n = 1 ist under Ar Phenyl oder durch Alkyl, Alkoxy, Dialkylamino, Cl oder Br substituiertes Phenyl ist und X Hydroxy oder Alkoxy ist und $R^1$ und $R^2$ unsubstituiertes Alkyl darstellen,

als Initiatoren für die Photopolymerisation ungesättigter Verbindungen sowie für die photochemische Vernetzung von Polyolefinen.

Es handelt sich bei diesen Verbindungen also um aromatisch-aliphatische Ketone, deren $\alpha$-C-Atomen tertiär ist und durch eine Hydroxyl- oder Aminogruppe oder deren Verätherungs- oder Silylierungsprodukt substituiert sind. Dabei kann der aliphatische Rest auch cycloaliphatisch oder araliphatisch sein oder mit dem aromatischen Rest unter Ringschluss verknüpft sein, was den benzocyclischen Ketonen der Formel IV entspricht.

Von den oben angeführten Substituenten können $R^1$, $R^2$ und $R^{10}$ Alkyl mit 1—8 C-Atomen sein, was z.B. Methyl, Aethyl, Propyl, Butyl, Hexyl oder Octyl sein kann. $R^4$, $R^5$ und $R^6$ als Alkyl können unverzweigtes oder verzweigtes Alkyl mit 1—12 C-Atomen sein wie z.B. Methyl, Aethyl, Isopropyl, Tert.Butyl, Isoamyl, n-Hexyl, 2-Aethylhexyl, n-Decyl oder n-Dodecyl. Alk bedeutet einen Niederalkylrest mit 1—4 C-Atomen wie z.B. Methyl, Aethyl, Isopropyl, n-Butyl oder tert.Butyl.

$R^1$ und $R^2$ in der Bedeutung von Hydroxyalkyl, Alkoxyalkyl oder Acyloxyalkyl können z.B. Hydroxymethyl, 1-Hydroxyäthyl, 2-Hydroxyäthyl, 2-Isopropoxyäthyl, 1-Hydroxyisobutyl, 1-Acetyloxybutyl, 1-Acryloyloxyhexyl, 1-Hydroxyoctyl, 3-Benzolyloxypropyl, Methoxymethyl oder Isobutyloxymethyl sein. Der Acylrest kann dabei der Rest einer aliphatischen oder aromatischen Carbonsäure sein. Bevorzugt handelt es sich um 1-Hydroxyalkylreste und deren Aether bzw. Ester. $R^4$, $R^5$ und $R^6$ als Hydroxyalkyl oder Alkoxyalkyl können z.B. 2-Hydroxyäthyl, 2-Butoxyäthyl, 2-Methoxypropyl, 3-Hydroxypropyl oder 2-Aethoxybutyl sein. Bevorzugt handelt es sich dabei um 2-Hydroxyalkylreste und deren Aether.

$R^1$ und $R^2$ als durch CN substituiertes Alkyl kann z.B. Cyanomethyl, 2-Cyanoäthyl, 2-Cyanopropyl, 4-Cyanobutyl, 2-Cyanohexyl oder 4-Cyanooctyl sein. Bevorzugt ist der 2-Cyanoäthylrest.

$R^1$ und $R^2$ als durch —COOAlk substituiertes Alkyl kann z.B. —$CH_2COOC_2H_5$, —$CH_2CH_2COOCH_3$, —$(CH_2)_3$—$COOCH_3$ oder —$CH_2$—$CH(C_2H_5)$—$COOC_4H_9$ sein.

$R^1$ und $R^2$ in der Bedeutung von Cycloalkyl können Cyclopentyl oder Cyclohexyl sein. $R^1$, $R^2$, $R^4$ und $R^5$ in der Bedeutung von Phenylalkyl können z.B. Benzyl, Phenyläthyl oder Dimethylbenzyl sein.

Ar als Aryl oder substituiertes Phenyl kann z.B. Phenyl, Naphthyl, Phenantryl, Anthracyl, Chlorphenyl, Bromphenyl, Dichlorphenyl, Mesityl, Isopropylphenyl, Phenoxyphenyl, Cyanophenyl, p-Nonylphenyl, Hydroxyphenyl, Tolyl, tert.Butylphenyl, Xylyl, Isopropyl-chlorphenyl, Methoxyphenyl, Aethoxyphenyl, Chlortolyl, Bromxylyl, Methylthiophenyl, Phenylthiophenyl, Butylsulfophenyl, Phenylsulfophenyl, Aethoxycarbonylphenyl, tert.Butoxycarbonylphenyl, Methylaminosulfophenyl, Dipropylaminosulfophenyl, Dimethylaminophenyl, Benzoylaminophenyl oder Acetylaminophenyl sein.

$R^1$ und $R^2$ zusammen können Alkylen oder Oxa- oder Azaalkylen bedeuten. In diesem Fall bilden $R^1$ und $R^2$ zusammen mit dem Kohlenstoffatom, an das sie gebunden sind, einen Cyclopropan-, Cyclobutan-, Cyclopentan-, Cyclohexan-, Cycloheptan, Tetrahydrofuran-, Tetrahydropyran-, Pyrrolidin- oder Piperidin-ring.

$R^2$ und $R^5$ zusammen können Alkylen oder Phenylalkylen mit 1 bis 9 Kohlenstoffatomen oder Oxa- oder Azaalkylen bedeuten. In diesem Fall bilden $R^2$ und $R^5$ zusammen mit dem Kohlenstoffatom, an das $R^2$ gebunden ist, und dem N-Atom, an das $R^5$ gebunden ist, einen 3- bis 6-gliedrigen Ring wie z.B. einen Aziridin-, Azetidin-, Pyrrolidin-, Imidazolidin, Piperidin-, Piperazin- oder Morpholinring.

$R^4$ und $R^5$ zusammen können Alkylen mit 4—5 C-Atomen bedeuten, das durch —O— oder —NH— oder —NAlk— unterbrochen sein kann. In diesem Fall bilden $R^4$ und $R^5$ zusammen mit dem N-Atom, an das sie gebunden sind, einen Pyrrolidin-, Piperidin-, Morpholin-, Piperazin- oder 4-Alkylpiperazin-ring. X und $R^1$ zusammen können eine Gruppe $—OCH(C_1—C_8\text{-Alkyl})—O(CH_2)_{1-2}—$, $—OCH(C_6—C_{14})—O(CH_2)_{1-2}—$, $—OCH(C_1—C_8\text{-Alkyl})—$ oder $—OCH(C_6—C_{14}\text{-Aryl})—$ darstellen. In diesem Fall bilden $R^1$ und X zusammen mit dem C-Atom, an das sie gebunden sind, einen Oxiran, 1,3-Dioxolan- oder 1,3-Dioxan-ring, der durch Alkyl oder Aryl substituiert sein kann.

$R^3$ kann Alkylen mit 1—6 C-Atomen sein, $R^{11}$ Alkylen mit 2—8 C-Atomen. Beispiele hierfür sind — innerhalb der angegebenen Anzahl der C-Atome — Methylen, 1,2-Aethylen, 1,3-Propylen, 1,4-Butylen, 2,2-Dimethyl-1,3-propylen, 1,6 Hexylen, 2-Methyl-3-äthyl-1,4-butylen oder 1,8-Octylen. $R^3$ kann auch Oxaalkylen, Thiaalkylen und Mono- oder Dioxothiaalkylen mit 2—6 C-Atomen sein wie z.B. 2-Oxa-1,3-propylen, 3-Oxa-2,4-pentylen, 3-Oxa-1,5-pentylen, $—CH_2SCH_2—$, $—CH_2CH_2SOCH_2CH_2—$ oder $—(CH_2)_3—SO_2—(CH_2)_3—$.

Ar kann Arylen mit 6—12 C-Atomen bedeuten wie z.B. Phenylen, Naphthylen oder Diphenylen.

Die Verbindungen der Formel IV stellen, wenn Y eine direkte Bindung ist, Derivate von Indanon, Cumaranon oder Thiocumaranon dar. Wenn Y $CH_2$ ist, so handelt es sich um Derivate von Tetralon Chromanon oder Thiochromanon.

Ein besonderer Gegenstand der Erfindung ist die Verwendung von Verbindungen der Formeln I oder II, worin X eine Gruppe $—NR^4R^5$ ist. Diese Verbindungen stellen am $\alpha$-C-Atom verzweigte und durch Aminogruppen substituierte Aryl-alkyl-Ketone dar.

Ein zweiter besonderer Gegenstand der Erfindung ist die Verwendung von Verbindungen der Formeln I oder II, worin X eine Gruppe $OR^6$ bedeutet. Diese Verbindungen stellen in $\alpha$-Stellung verzweigte und durch Hydroxyl- oder Aethergruppen substituierte Aryl-alkyl-Ketone dar.

Bevorzugt ist die Verwendung von Verbindungen der Formeln I, II, III oder IV, worin n 1 oder 2 ist, Ar, wenn n = 1 ist, Aryl mit 6—14 C-Atomen oder durch Cl, Br, Alk, —OAlk, —OPhenyl, —COOAlk, $—N(Alk)_2$ oder —NHCOAlk substituiertes Phenyl darstellt und Alk einen Niederalkylrest mit 1—4 C-Atomen bedeutet und wenn n = 2 ist, $C_6—C_{12}$ Arylen oder eine Gruppe -Phenylen-T-Phenylen- bedeutet, X eine der Gruppen $—NR^4R^5$ oder $—OR^6$ darstellt, X' eine der Gruppen

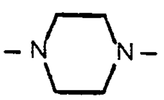,

$—N(R^{10})—R^{11}—N(R^{10})—$ oder $—O—R^{12}—O—$ darstellt, Y eine direkte Bindung oder $—CH_2—$ darstellt, Z —O—, $—CH_2$ oder $—C(CH_3)_2—$ darstellt, T —O— oder $—CH_2—$ darstellt, $R^1$ in Formel I bei n = 1 und X = $—OR^6$ unsubstituiertes oder durch —COOAlk oder CN substituiertes $C_1—C_8$ Alkyl, oder $C_7—C_9$ Phenylalkyl bedeutet, und in allen anderen Fällen unsubstituiertes oder durch —OH, OAlk, —COOAlk oder —CN substituiertes $C_1—C_8$ Alkyl, oder $C_7—C_9$ Phenylalkyl bedeutet, $R^2$ eine der für $R^1$ gegebenen Bedeutungen hat oder Allyl oder eine Gruppe $—CH_2CH_2R^{13}$ ist, oder zusammen mit $R^1$ $C_4—C_6$ Alkylen oder $C_3—C_4$ Oxa- oder Azaalkylen bedeutet, $R^3$ eine direkte Bindung oder $C_1—C_6$ Alkylen bedeutet oder zusammen mit den beiden Substituenten $R^2$ und den beiden C-Atomen, an die diese Substituenten gebunden sind, einen Cyclopentan- oder Cyclohexanring bildet, $R^4$ $C_1—C_{12}$ Alkyl, durch OH, OAlk oder CN substituiertes $C_2—C_4$ Alkyl oder Allyl bedeutet, $R^5$ $C_1—C_{12}$ Alkyl, durch OH oder OAlk substituiertes $C_2—C_4$ Alkyl oder Allyl bedeutet oder zusammen mit $R^4$ $C_4—C_5$ Alkylen bedeutet, das durch —O— oder —NAlk— unterbrochen sein kann, $R^6$ Wasserstoff, $C_1—C_{12}$ Alkyl, durch OH oder OAlk substituiertes $C_2—C_4$ Alkyl, Allyl, Benzyl oder Phenyl bedeutet, $R^{10}$ $C_1—C_8$ Alkyl, $R^{11}$ $C_2—C_8$ Alkylen und $R^{13}$ $—CONH_2$, —CONHAlk, $—CON(Alk)_2$, $—P(O)(OAlk)_2$ oder 2-Pyridyl bedeuten.

Unter diesen Verbindungen sind die Verbindungen der Formeln I, II oder III bevorzugt, worin n 1 oder 2 ist, Ar wenn n = 1 ist, $C_6C_{14}$-Aryl oder durch Cl, Br, Alk oder OAlk substituiertes Phenyl darstellt und Alk einen Niederalkylrest mit 1—4 C-Atomen bedeutet und wenn n = 2 ist, $C_6—C_{12}$ Arylen, oder eine Gruppe -Phenylen-T-Phenylen- bedeutet, X eine der Gruppen $—NR^4R^5$ oder $—OR^6$ darstellt, X' die Gruppe

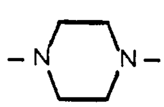

darstellt T —O— oder $—CH_2—$ darstellt, $R^1$ $C_1—C_8$ Alkyl bedeutet, $R^2$ $C_1—C_8$ Alkyl oder Allyl ist, $R^3$ eine direkte Bindung oder $C_1—C_6$ Alkylen ist, $R^4$ $C_1—C_{12}$ Alkyl bedeutet, $R^5$ $C_1—C_{12}$ Alkyl bedeutet oder zusammen mit $R^4$ $C_4—C_5$ Alkylen bedeutet, das durch —O— oder —NAlk— unterbrochen sein kann und $R^6$ Wasserstoff, $C_1—C_{12}$ Alkyl, durch OH oder OAlk substituiertes $C_2—C_4$ Alkyl, Allyl, Benzyl oder Phenyl bedeutet.

Bevorzugt ist weiterhin die Verwendung von Verbindungen der Formel I, worin n = 1 und X = OH ist, $R^1$ und $R^2$ zusammen Alkylen mit 2—5 C-Atomen darstellen und Ar die oben gegebene Bedeutung hat,

sowie die Verwendung von Verbindungen der Formel I, worin n = 2 ist und Ar, $R^1$, $R^2$ und X die oben gegebene Bedeutung haben.

Beispiele für erfindungsgemäss verwendbare Verbindungen der Formel I, worin n = 1 ist, sind folgende:

2-Hydroxy-2-methyl-(p-phenoxypropiophenon)
2-Hydroxy-2-methyl-(p-acetylaminopropiophenon)
2-Hydroxy-2-methyl-(p-methylthio-propiophenon)
2-Hydroxy-2-methyl-(p-carbäthoxy-propiophenon)
2-Phenoxy-2-methyl-propiophenon
2-Methylthiomethoxy-2-methyl-propiophenon
2-Allyloxy-2-methyl-propiophenon
2-Benzyloxy-2-methyl-propiophenon
2-Methoxyäthoxy-2-methyl-propiophenon
2-Hydroxymethoxy-2-methyl-propiophenon
2-Hydroxyäthoxy-2-methyl-propiophenon
2-Methyl-2-piperidino-3-phenyl-3-hydroxy-propiophenon
2-Methyl-2-morpholino-3-phenyl-3-hydroxy-propiophenon
2-Methyl-2-dimethylamino-3-phenyl-3-hydroxy-propiophenon
$\alpha$-Hydroxy-$\alpha$-$\alpha$-bis-(cyanäthyl)-acetophenon
$\gamma$-Hydroxy-$\gamma$-benzoylpimelinsäure-diäthylester
2-Di-(2-hydroxyäthyl)-amino-2-methyl-3-phenyl-propiophenon
2-Dimethylamino-2-methyl-propiophenon
2-Diäthylamino-2-methyl-propiophenon
2-Dibutylamino-2-methyl-propiophenon
2-Di-hydroxyäthylamino-2-methyl-propiophenon
2-Piperidino-2-methyl-propiophenon
2-(2-Methylpiperidino)-2-methyl-propiophenon
2-Morpholino-2-methyl-propiophenon
2-Piperazino-2-methyl-propiophenon
2-(4-Methylpiperazino)-2-methyl-propiophenon
2-Pyrrolidino-2-methyl-propiophenon
2-Methylphenylamino-2-methyl-propiophenon
1-Benzoyl-cyclohexanol
1-Benzoyl-cyclopentanol
3-p-Methoxybenzoyl-3-dimethylaminoheptan
2-(2-Methoxybenzoyl)-2-diallylaminopropan
2-(2-Thenoyl)-2-piperidinopropan
2-p-Phenylbenzoyl-2-di-(2-hydroxyäthyl)-aminopropan
2-o-Toluyl-2-(trimethylsiloxy)-propan
2-Hydroxymethoxy-2-methyl-propiophenon
2-Hydroxymethoxy-2-methyl-2,5-dimethylpropiophenon)
2-Hydroxymethoxy-2-methyl-(p-isopropylpropiophenon)
5-Methyl-5-benzoyl-1,3-dioxolan
2,5-Dimethyl-5-benzoyl-1,3-dioxolan
2-Phenyl-5-methyl-5-benzoyl-1,3-dioxolan
5-Methyl-5-(p-isopropylbenzoyl)-1,3-dioxolan
2,3-Epoxy-2-methyl-3-phenyl-propiophenon
2-Acetoxymethoxy-2-methyl-propiophenon
2-Benzoyloxymethoxy-2-methyl-propiophenon
2-Hydroxy-2-methyl-4,-N,N-diäthylcarbamoyl-butyrophenon
2-Morpholino-2-methyl-4-N,N-diäthylcarbamoylbutyrophenon
2-Hydroxy-2-methyl-4-(2-pyridyl)-butyrophenon
2-Hydroxy-2-methyl-4-diäthylphosphono-butyrophenon
2-Hydroxy-2-benzyl-propiophenon
2-Hydroxy-2-(p-methylbenzyl)-propiophenon
2-Hydroxy-2-cyclohexyl-propiophenon
2-Hydroxy-2-cyclopentyl-propiophenon
2-(2-Hydroxyäthoxy)-2-methyl-propiophenon
2-Hydroxy-2-allyl-propiophenon
2-Hydroxy-2-methyl-4-(2-oxo-1-pyrrolidinyl)-butyrophenon

Beispiele für Verbindungen der Formel I, worin n = 2 ist, sind:

4,4'-Bis-($\alpha$-hydroxy-isobutyryl)-diphenyloxid

4,4'-Bis-(α-hydroxy-isobutyryl)-diphenyl
4,4'-Bis-(α-hydroxy-isobutyryl)-diphenylsulfid
4,4'-Bis-(α-hydroxy-isobutyryl)-diphenylmethan
4,4'-Bis-(α-piperidino-isobutyryl)-diphenyloxyid
4,4'-Bis-[α-(isopropylamino)-isobutyryl]-diphenyl
4,4'-Bis-(α-benzoyloxy-isobutyryl)-diphenyloxid.
4,4'-Bis-(α-hydroxy-isobutyryl)-diphenyläthan

Beispiele für Verbindungen der Formel II sind:

1,4-Diphenyl-2,3-dimethyl-2,3-dihydroxy-butandion-1,4
2,4-Dibenzoyl-2,4-dihydroxy-pentan
2,9-Dibenzoyl-2,9-dimethyl-3,8-dioxadecan
2,7-Dibenzoyl-2,7-dimethyl-3,6-dioxaoctan
1,6-Diphenyl-2,5-dimethyl-2,5-dihydroxy-hexandion-1,6
1,4-Diphenyl-2,3-dimethyl-2,3-bis-(dimethylamino)-butandion-1,4
1,4-Diphenyl-2,3-dimethyl-2,3-dipiperidyl-butandion-1,4
1,2-Bis-hydroxy-1,2-bis-benzoyl-cyclohexan
1,2-Bis-dimethylamino-1,2-bis-benzoyl-cyclohexan
1,2-Bis-morpholino-1,2-bis-benzoyl-cyclohexan
Bis-(3-hydroxy-3-benzoylbutyl)-sulfon

Beispiele für Verbindungen der Formel III sind:

1,4-Bis-(1-benzoyl-isopropyl)-piperazin
2,7-Dibenzoyl-2,7-dimethyl-3,6-dioxaoctan
2,9-Dibenzoyl-2,9-dimethyl-3,8-dioxadecan
2,6-Dibenzoyl-2,6-dimethyl-3,5-dioxaheptan
N,N-Bis-(α-benzoyl-isopropyl)-butylamin
N,N'-Dimethyl-N,N'-bis(α-benzoyl-isopropyl)-äthylendiamin

Beispiele für Verbindungen der Formel IV sind:

1-Oxo-2-dimethylamino-2-methyl-1,2,3,4-tetrahydronaphthalin
1-Oxo-2-hydroxy-2-methyl-1,2,3,4-tetrahydronaphthalin
1-Oxo-2-hydroxy-2-methylindan.

Die Verbindungen der Formeln I, II, III und IV sind zum Teil bekannte Verbindungen, zum Teil handelt es sich um neue Verbindungen.

Bekannt sind Verbindungen der Formel I, worin n = 1 ist, Ar Phenyl, durch Methyl oder Methoxy substituiertes Phenyl oder Furyl ist, $R^1$ und $R^2$ Methyl sind oder $R^1$ und $R^2$ zusammen Alkylen sind und X Hydroxy, Methoxy oder Nitrophenoxy ist.

Bekannt sind Verbindungen der Formel I, worin n = 1 ist, Ar Phenyl, Chlorphenyl oder Diphenylyl ist, $R^1$ und $R^2$ Methyl oder Morpholinomethyl sind oder $R^1$ und $R^2$ zusammen Alkylen sind und X eine Gruppe —$NR^5R^6$ ist, wobei $R^5$ und $R^6$ Alkyl oder Benzyl sind oder $R^5$ und $R^6$ zusammen Alkylen oder Oxaalkylen bedeuten.

Bekannt ist ferner eine Verbindung der Formel II, worin Ar Phenyl, $R^1$ Methyl, X Hydroxy und $R^3$ eine direkte Bindung ist.

Die bekannten Verbindungen wurden bisher nicht als Photoinitiatoren vorgeschlagen.

Die Erfindung betrifft auch die Verbindungen der Formeln I, II, III oder IV, soweit sie neu sind. Gegenstand der Erfindung sind daher auch

a) Verbindungen der Formel I, worin n = 1 ist, Ar $C_{10}$—$C_{14}$ Aryl, durch CN, OH, —OPhenyl, —SAlk, —$SO_2$Alk, —$SO_2$Phenyl, —COOAlk, —$SO_2NH_2$, —$SO_2$NHAlk, —$SO_2N(Alk)_2$, —NHAlk, oder —NHCOAlk substituiertes Phenyl, Thienyl oder Pyridyl ist, X OH oder —OAlk ist worin Alk einen $C_1$—$C_4$-Niederalkylrest bedeutet und $R^1$ und $R^2$ die eingangs gegebenen Bedeutung haben.

b) Verbindungen der Formel I, worin n = 1 ist, X eine Gruppe —$OR^6$, und $R^6$ durch OH oder OAlk substituiertes $C_2$—$C_4$ Alkyl, Allyl, Cyclohexyl, Benzyl, unsubstituiertes oder durch Cl oder Alk substituiertes Phenyl bedeutet, worin Alk einen $C_1$—$C_4$-Alkylrest bedeutet und Ar, $R^1$ und $R^2$ die eingangs gegebene Bedeutung haben.

c) Verbindungen der Formel I, worin n = 1 ist, X eine Gruppe —$OSiR^7(R^8)_2$ darstellt, $R^7$ $C_2$—$C_4$ Alkyl oder Phenyl ist und Ar, $R^1$, $R^2$ und $R^8$ die eingangs gegebene Bedeutung haben.

d) Verbindungen der I, worin n = 1 ist, Ar Phenyl, Halogenphenyl oder Diphenylyl ist, X eine Gruppe —$NR^4R^5$ ist, $R^1$ $C_2$—$C_8$Alkyl, durch OH, OAlk, $C_2$—$C_8$ Acyloxy, —COOAlk oder CN substituiertes $C_1$—$C_8$Alkyl, $C_5$—$C_6$Cycloalkyl oder $C_7$—$C_9$ Phenylalkyl ist, $R^2$ eine der für $R^1$ angegebenen Bedeutung hat oder Allyl ist oder zusammen mit $R^1$ $C_4$—$C_6$ Alkylen oder $C_3$—$C_4$ Oxa- oder Azaalkylen bedeutet und $R^4$ und $R^5$ die eingangs gegebene Bedeutung haben.

e) Verbindungen der Formel I, worin n = 1 ist, Ar durch CN, Alk, OH, OAlk, —OPhenyl, —SAlk, —$SO_2$Alk, —$SO_2$Phenyl, —COOAlk, —$SO_2NH_2$, —$SO_2$NHAlk, —$SO_2N(Alk)_2$, —NHAlk, —$N(Alk)_2$ oder —NHCOAlk substituiertes Phenyl oder Naphthyl, Thienyl, Pyridyl oder Furyl ist, Alk eine $C_1$—$C_4$-Alkylgruppe bedeutet, X eine Gruppe —$NR^4R^5$ darstellt und $R^1$,$R^2$,$R^4$ unf $R^5$ die eingangs gegebene Bedeutung haben.

f) Verbindungen der Formel I, worin n = 2 ist und Ar, X, $R^1$ und $R^2$ die eingangs gegebene Bedeutung haben.

Diese neuen Verbindungen können in Analogie zu den bekannten Verbindungen hergestellt werden, wofür verschiedene Methoden in Frage kommen.

So können die Verbindungen der Formel I aus aromatisch-aliphatischen Ketonen durch folgende Reaktionsstufen hergestellt werden:

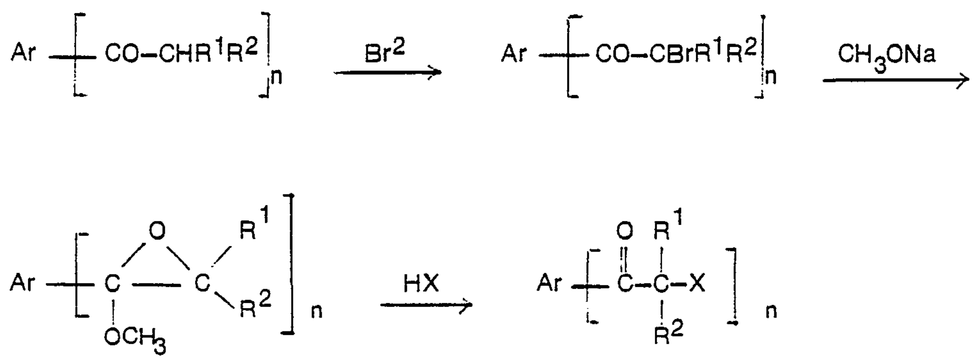


Als HX können dabei Amine verwendet werden [C.L. Stevens, Ch. Hung Chang, J. Org. Chem *27* (1962), 4392] oder Wasser [C.L. Stevens, E. Farkas, J. Am. Chem. Soc. *74* (1952), 618 und C.L. Stevens, S.J. Dykstra, J. Am. Chem. Soc. *75* (1953), 5976].

In vielen Fällen gelingt auch die direkte Umsetzung der $\alpha$-Bromketone zu Verbindungen der Formel I,

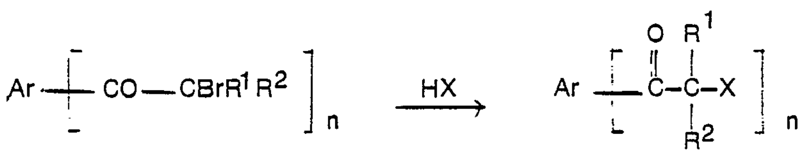


I

beispielsweise mit Aminen, Alkalihydroxiden oder Alkaliphenoxiden. Anstelle von Bromverbindungen können auch die entsprechenden Chlorverbindungen verwendet werden.

Die so erhältlichen Hydroxyketone der Formel I (X = OH) können nach den üblichen Methoden veräthert oder O-silyliert werden.

Verwendet man bei den oben angegebenen Reaktionen anstelle der monofunktionellen Verbindung HX eine difunktionelle Verbindung H—X'—H, so erhält van Verbindungen der Formel III.

Die Verbindungen der Formel II können analog denjenigen der Formel I hergestellt werden, indem man als Ausgangsmaterial Diketone der allgemeinen Formel

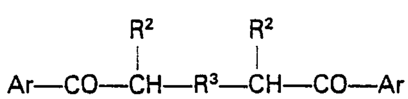

verwendet. Ebenfalls in analoger Weise erhält man die Verbindungen der Formel IV aus cyclischen Ketonen der Formel

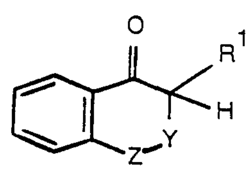

als Ausgangsmaterial.

Verbindungen, der Formel I, in denen $R^1$ eine substituierte Alkylgruppe ist können aus den Verbindungen der Formel $Ar\!-\![CO—CH(R^2)—X]_n$ durch Reaktion mit Aldehyden ($R^1$ = Hydroxyalkyl) oder mit einer additionsfähigen Vinylverbindung, z.B. mit Acrylsäureestern oder Acrylnitril gewonnen werden. In derselben Weise kann eine Gruppe $—CH_2CH_2—R^{13}$ als $R^2$ eingeführt werden, wobei man von einer Verbindung $Ar\!-\![CO—CH(R^1)—X]_n$ ausgeht. Wenn sowohl $R^1$ wie $R^2$ substituiertes Alkyl ist, so können beide Substituenten gemeinsam eingeführt werden durch Reaktion einer Verbindung $Ar\!-\![CO—CH_2—X]_n$ mit mindestens 2 Mol eines Aldehydes oder einer Vinylverbindung. Aus den Hydroxyalkylgruppen $R^1$ und/oder $R^2$ lassen sich durch Verätherung oder Veresterung die entsprechenden Alkoxyalkyl- und Acyloxyalkylgruppen erhalten. Analog lassen sich auch Verbindungen der Formel II, III und IV mit substituierten Alkylgruppen als $R^1$ oder $R^2$ herstellen.

Während alle diese aufgezählten Synthesemethoden von einem aromatisch-aliphatischen Keton ausgehen, in das auf verschiedene Weise ein Substituent X eingeführt wird, ist es in bestimmten Fällen auch möglich, den Substituenten X schon während der Keton-Synthese nach Friedel-Crafts einzuführen nach dem Reaktionsschema:

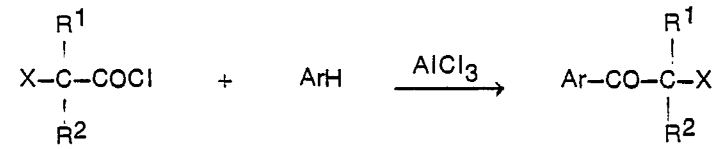


Dies setzt voraus, dass der Substituent X unter den Reaktionsbedingungen der Friedel-Crafts-Reaktion nicht angegriffen wird. Auf diese Weise lassen sich z.B. bei Verwendung heterocyclischer Carbonsäurechloride Verbindungen der Formel I herstellen, in denen X und $R^1$ zusammen mit dem C-Atom, an das sie gebunden sind, einen Heteroring bilden.

Erfindungsgemäss können die Verbindungen der Formel I, II, III und IV als Initiatoren zur Photopolymerisation von ungesättigten Verbindungen oder Systemen, die solche Verbindungen enthalten, verwendet werden.

Solche Verbindungen sind beispielsweise ungesättigte Monomere wie Ester von Acryl- oder Methacrylsäure, z.B. Methyl-, Aethyl-, n- oder tert.Butyl-, Isooctyl- oder Hydroxyäthylacrylat, Methyl- oder Aethylmethacrylat, Aethylendiacrylat, Neopentyl-diacrylat, Trimethylolpropantrisacrylat, Pentareythrit-tetraacrylat oder Pentaerythrittrisacrylat; Acrylnitril, Methacrylnitril, Acrylamid, Methacrylamid, N-substituierte (Meth)acrylamide; Vinylester wie z.B. Vinylacetat, -propionat, -acrylat oder -succinat; sonstige Vinylverbindungen wie Vinyläther, Styrol, Alkylstyrole, Halogenstyrole, Divinylbenzol, Vinylnaphthalin, N-Vinylpyrrolidon, Vinylchlorid oder Vinylidenchlorid; Allylverbindungen wie Diallylphthalat, Diallylmaleat, Triallylisocyanurat, Triallylphosphat oder Aethylenglycol-diallyläther und die Mischungen von solchen ungesättigten Monomeren.

Photopolymerisierbare Verbindungen sind weiterhin ungesättigte Oligomere oder Polymere und deren Mischungen mit ungesättigten Monomeren. Hierzu zählen thermoplastische Harze, die ungesättigte Gruppen wie Fumarsäureester, Allylgruppen oder Acrylat- oder Methacrylatgruppen enthalten. Meist sind diese ungesättigten Gruppen über funktionelle Gruppen an die Haputkette dieser linearen Polymeren gebunden. Grosse Bedeutung haben Gemische von Oligomeren mit einfach und mehrfach ungesättigten Monomeren. Beispiele für solche Oligomere sind ungesättigte Polyester, ungesättigte Acrylharze und Isocyanat- oder Epoxid- modifizierte Acrylatoligomere sowie Polyätheracrylatoligomere. Beispiele für mehrfach ungesättigte Verbindungen sind vor allem die Acrylate von Diolen und Polyolen, z.B. Hexamethylen-diacrylat oder Pentaerythrit-tetracrylat. Auch als einfach ungesättigte Monomere werden Acrylate bevorzugt wie z.B. Butylacrylat Phenylacrylat, Benzylacrylat, 2-Aethylhexylacrylat oder 2-Hydroxypropylacrylat. Man hat es durch Auswahl aus den verschiedenen Vertretern der drei Komponenten in der Hand, die Konsistenz des unpolymerisierten Gemisches sowie die Plastizität des polymerisierten Harzes zu variieren.

Neben diesen Dreikomponenten-Gemischen spielen bei den Polyesterharzen vor allem Zweikomponenten-Gemische eine grosse Rolle. Diese bestehen meist aus einem ungesättigten Polyester und einer Vinylverbindung. Die ungesättigten Polyester sind oligomere Veresterungsprodukte mindestens einer ungesättigten Dicarbonsäure wie z.B. Malein-, Fumar- oder Citraconsäure, und meist mindestens einer gesättigten Dicarbonsäure, wie z.B. Phthalsäure, Bernsteinsäure, Sebazinsäure oder Isophthalsäure, mit Glykolen wie z.B. Aethylenglykol, Propandiol-1,2, Di- oder Triäthylenglykol oder Tetramethylenglykol, wobei zur Modifizierung meist auch Monocarbonsäuren und Monoalkohole mitverwendet werden. Diese ungesättigten Polyester werden üblicherweise in einer Vinyl- oder Allylverbindung gelöst, bevorzugt wird hierfür Styrol verwendet.

Photopolymerisierbare Systeme, wie sie für die verschiedenen Zwecke verwendet werden, enthalten meist ausser den photopolymersierbaren Verbindungen und dem Photoinitiator eine Reihe sonstiger Zusätze. So ist es vielfach üblich, thermische Inhibitoren zuzusetzen, die vor allem während der Herstellung der Systeme durch Mischen der Komponenten vor einer vorzeitigen Polymerisation schützen sollen. Hierzu werden beispielsweise Hydrochinon, Hydrochinonderivate, p-Methoxyphenol, $\beta$-Naphthylamin oder $\beta$-Naphthole verwendet. Weiter können geringe Mengen von UV-Absorben zugesetzt werden wie z.B. solche vom Benztriazol- oder Benzophenontyp.

Zur Erhöhung der Dunkellagerstabilität können Kupferverbindungen wie Kupfernaphthenat, -stearat, oder -octoat, Phosphorverbindungen wie Triphenylphosphin, Tributylphosphin, Triäthylphosphit, Triphenylphosphit oder Tribenzylphosphat, quartäre Ammoniumverbindungen wie Tetramethylammoniumchlorid oder Trimethyl-benzylammoniumchlorid oder Hydroxylaminderivate wie z.B. N-Diäthylhydroxylamin, zugesetzt werden. Weiterhin können die photopolymerisierbaren Systeme Kettenübertragungsmittel wie z.B. N-Methyldiäthanolamin, Triäthanolamin oder Cyclohexen enthalten.

Um die inhibierende Wirkung des Luftsauerstoffs auszuschliessen setzt man photohärtbaren Systemen häufig Paraffin oder ähnliche wachsartige Stoffe zu. Diese schwimmen bei Beginn der Polymerisation wegen mangelnder Löslichkeit im Polymeren aus und bilden eine transparente Oberflächenschicht, die den Zutritt von Luft verhindert. Auch durch Einführung von autoxydablen Gruppen, beispielsweise Allylgruppen, in das zu härtende Harz kann der Luftsauerstoff desaktiviert werden.

Die Photoinitiatoren können auch in Kombination mit radikalischen Initiatoren wie z.B. Peroxiden, Hydroperoxiden, Keton-peroxiden oder Percarbonsäureestern verwendet werden.

Photopolymerisierbare Systeme enthalten weiterhin — je nach Verwendungszweck — Füllstoffe wie Kieselsäure, Talkum oder Gips, Pigmente, Farbstoffe, Fasern, Thixotropiemittel oder Verlaufhilfsmittel.

Ferner können auch Kombinationen mit bekannten Photoinitiatoren, wie Benzoinäthern, Dialkoxyacetophenonen oder Benzilketalen, verwendet werden.

Besonders für die Photopolymerisation von dünnen Schichten und Druckfarben können Kombinationen der erfindungsgemässen Photoinitiatoren mit Aminen und/oder aromatischen Ketonen verwendet werden. Beispiele für Amine sind Triäthylamin, N-Methyldiäthanolamin, N-Dimethyläthanolamin oder p-Dimethylaminobenzoesäureester. Beispiele für Ketone sind Benzophenon, substituierte Benzophenonderivate, Michler's Keton, Antrachinon und Anthrachinonderivate, sowie Thioxanthon und dessen Derivate.

Grosse Bedeutung hat die Photohärtung für Druckfarben, da die Trocknungszeit des Bindemittels ein massgeblicher Faktor für die Produktionsgeschwindigkeit graphischer Erzeugnisse ist und in der Grössenordnung von Bruchteilen von Sekunden liegen soll. Gut geeignet sind die erfindungsgemässen Initiatoren auch für photohärtbare Systeme zur Herstellung von Druckplatten. Hierbei werden z.B. Gemische von löslichen linearen Polyamiden mit photopolymerisierbaren Monomeren, beispielsweise Acrylamide, und einem Photoinitiator verwendet. Filme oder Platten aus diesen Systemen werden über das Negativ (oder Positiv) der Druckvorlage belichtet und die ungehärteten Anteile anschliessend mit einem Lösungsmittel eluiert.

Ein weiteres Einsatzgebiet der UV-Härtung ist die Metallbeschichtung, beispielsweise bei der Lackierung von Blechen für Tuben, Dosen oder Flaschenverschlüssen, sowie die UV-Härtung von Kunstoffbeschichtungen, beispielsweise von Fussboden- oder Wandbelägen auf PVC-Basis.

Beispiele für die UV-Härtung von Papierbeschichtungen sind die farblose Lackierung von Ettiketten, Schallplatten-Hüllen oder Buchumschlägen.

Erfindungsgemäss können die Verbindungen der Formeln I, II, III und IV auch als Initiatoren zur photochemischen Vernetzung von Polyolefinen verwendet werden. Hierfür kommen z.B. Polypropylen, Polybuten, Polyisobutylen sowie Copolymerisate wie z.B. Aethylen-Propylen-Copolymere in Frage, vorzugsweise jedoch Polyäthylen von niedrigen, mittlerer oder hoher Dichte.

Die Photoinitiatoren werden für die angeführten Anwendungsgebiete zweckmässig in Mengen von 0,1 bis 20 Gew.-%, vorzugsweise etwa 0,5 bis 5 Gew.-%, bezogen auf das photopolymerisierbare bzw. vernetzbare System, angewendet. Unter System ist hierbei das Gemisch aus der photopolymerisierbaren bzw. vernetzbaren Verbindung, dem Photoinitiator und den sonstigen Füll- und Zusatzstoffen gemeint wie es in der jeweiligen Anwendung verwendet wird.

Der Zusatz der Photoinitiatoren zu den photopolymerisierbaren Systemen geschieht im allgemeinen durch einfaches Einrühren, da die meisten dieser Systeme flüssig oder gut löslich sind. Meist kommt es zu einer Lösung der erfindungsgemässen Initiatoren, wodurch deren gleichmässige Verteilung sowie die Transparenz der Polymerisate gewährleistet ist.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, sowie superaktinische Leuchtstoffröhren geeignet, deren Emissionsmaxima im Bereich zwischen 250 und 400 nm liegen.

Die Herstellung und Verwendung der erfindungsgemässen Photoinitiatoren ist in den folgenden Beispielen näher beschrieben. Hieren bedeuten Teile Gewichtsteile, Prozente Gewichtsprozente und die Temperatur ist in Celsius-Graden angegeben.

Herstellung und Eigenschaften der in den Beispielen 1—6 verwendeten Verbindungen

Die Herstellung der in Tabelle 1 aufgeführten Verbindungen geschah nach einer oder mehreren der Methoden A bis L.

*Methode A* Chlorierung von aromatisch-aliphatischen Ketonen

$$Ar{-}[CO{-}CR^1R^2H]_n + n\ Cl_2 \rightarrow Ar{-}[CO{-}CR^1R^2Cl]_n + n\ HCl$$

Zur Durchführung wird das Keton in einem inerten Lösungsmittel, vorzugsweise in Tetrachlormethan, gelöst und bei 40—80°C die berechnete Menge Chlor in die Lösung eingeleitet. Dann wird zur Entfernung des gelösten HCl Stickstoff eingeleitet. Zum Schluss wird das Lösungsmittel abdestilliert. Eine Reinigung des erhaltenen Chlorketons ist meist nicht notwendig, das Produkt kann anschliessend nach Methode D, F oder H umgesetzt werden.

*Methode B* Bromierung von aromatisch-aliphatischen Ketonen

$$Ar{-}[CO{-}CR^1R^2H]_n + n\ Br_2 \rightarrow Ar{-}[CO{-}CR^1R^2Br]_n + n\ HBr$$

Zur Durchführung tropft man bei Raumtemperatur die berechnete Menge Brom in eine Lösung des Ketons, beispielsweise in $CCl_4$. Die Aufarbeitung und Weiterverarbeitung geschieht wie bei Methode A.

*Methode C* Chlorierung mit Sulfurylchlorid

$$Ar{-}[CO{-}CR^1R^2H]_n + n\ SO_2Cl_2 \rightarrow Ar{-}[CO{-}CR^1R^2{-}Cl]_n + n\ SO_2 + n\ HCl$$

Das Sulfurylchlorid wird bei 40°C der $CCl_4$-Lösung des Ketons zugetropft. Aufarbeitung und Weiterverarbeitung wie bei Methode A.

*Methode D* Herstellung der Epoxid-Zwischenprodukte

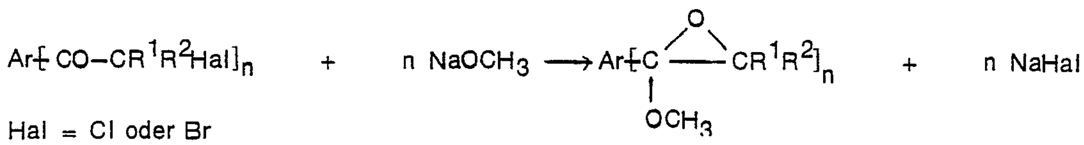

Hal = Cl oder Br

Das Halogenketon wird in Methanol gelöst und eine Lösung der stöchiometrischen Menge Natriummethoxid in Methanol wird bei Rückflusstemperatur zugetropft. Dann wird das Methanol abdestilliert, der Rückstand auf Eiswasser gegossen und mit Aether extrahiert. Die Aetherlösung wird mit Wasser gewaschen, über $Na_2SO_4$ getrocknet und eingedampft. Der Rückstand wird durch Umkristallisation oder Vakuum-Destillation gereinigt. Das Epoxid kann anschliessend nach Methode E oder G umgesetzt werden.

*Methode E* Hydrolyse der Epoxide

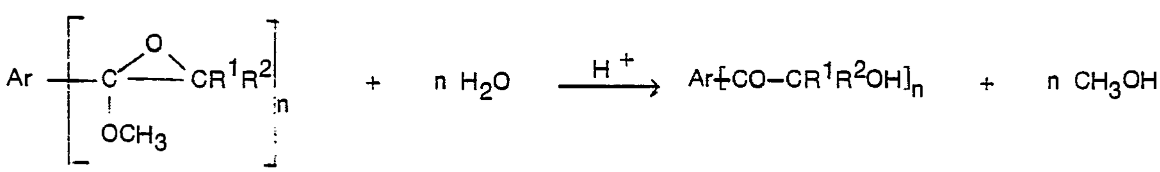

Das Epoxid wird mit der 2—5 fachen Gewichtsmenge Wasser übergossen und unter Zusatz einer katalytischen Menge Mineralsäure 1—2 Stunden unter Rückfluss gekocht. Nach dem Erkalten wird mit Aether extrahiert, die Aetherlösung mit Wasser gewaschen, über $Na_2SO_4$ getrocknet und eingedampft. Der Rückstand (rohes Hydroxyketon) wird durch Destillation oder Kristallisation oder Säulenchromatographie gereinigt. Die Eigenschaften der reinen $\alpha$-Hydroxyketone sind in Tabelle 1 angegeben.

*Methode F* $\alpha$-Hydroxyketone aus $\alpha$-Halogenketonen

$$Ar{-}[CO{-}CR^1R^2Hal]_n + n\ NaOH \rightarrow Ar{-}[CO{-}CR^1R^2OH]_n + n\ NaHal$$

Das $\alpha$-Halogenketon wird in verdünnter oder konzentrierter Natronlauge (20% Ueberschuss an NaOH) unter Rückfluss gekocht. Nach Beendigung der Hydrolyse (Kontrolle durch Chromatographie) wird das rohe Hydroxyketon wie in E beschrieben isoliert und gereinigt.

*Methode G* $\alpha$-Aminoketone aus den Epoxiden.

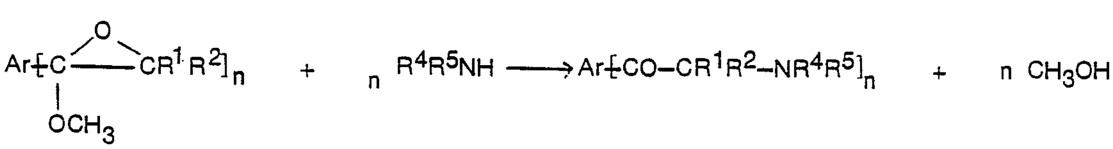

Das Epoxid wird entweder ohne Lösungsmittel oder unter zusatz von wenig Toluol oder Xylol mit der stöchiometrischen Menge des Amins vernetzt und etwa 10—20 Std. bei 100—200°C reagieren gelassen. Bei niedrig-siedenden Aminen, wie z.B. Dimethylamin oder Diäthylamin wird die Reaktion im autoklaven ausgeführt. Das Reaktionsgemisch wird mit Benzol verdünnt, mit verdünnter Salzsäure extrahiert. Die wässrigsaure Lösung wird mit NaOH alkalisch gemacht, mit Aether extrahiert, die Aetherlösung mit Wasser gewaschen, über $Na_2SO_4$ getrocknet und eingedampft. Das erhaltene Rohprodukt wird durch Destillation oder Kristallisation gereinigt. Die $\alpha$-Aminoketone sind in Tabelle 1 aufgeführt.

*Methode H* $\alpha$-Aminoketone aus den $\alpha$-Halogenketonen

$$Ar-[CO-CR^1R^2Hal]_n + 2n\ R^4R^5NH \rightarrow Ar-[CO-CR^1R^2-NR^4R^5]_n + n\ R^4R^5NH_2Hal$$

Das $\alpha$-Halogenketon wird unverdünnt oder mit Toluol verdünnt mit 2 Mol-Aequivalenten des Amins gemischt und 10—20 Std. auf 100—200°C erwärmt. Im Falle niedrig-siedender Amine wie z.B. Dimethylamin oder Diäthylamin wird die Umsetzung im Autoklaven durchgeführt. Isolierung und Reinigung geschieht wie bei Methode G.

*Methode I* Einführung einer Carbalkoxyäthylgruppe

$$Ar[CO-CHR^1-X]_n \quad + \quad n\ CH_2 = CH-COOAlk \longrightarrow Ar[CO-\underset{}{\overset{CH_2CH_2COOAlk}{\overset{|}{C}}}R^1-X]_n$$

Das Keton wird in Dimethylsulfoxid gelöst, dazu fügt man 1,1 Mol-Aequivalent NaOH in Form von 4 n Natronlauge und lässt bei Raumtemperatur unter Kühlung 1,1 Mol-Aequivalent Acrylester zutropfen. Das Reaktionsgemisch wird mit Eiswasser verdünnt und mit Toluol extrahiert. Die Toluollösung wird mit Wasser neutral gewaschen, über $Na_2SO_4$ getrocknet und eingedampft. Das Rohprodukt wird durch Säulenchromatographie oder Kristallisation gereinigt.

*Methode K* Verätherung von Hydroxyketonen

$$Ar-[CO-CR^1R^2-OH]_n + n\ R^6Hal + n\ NaOH \rightarrow Ar-[CO-CR^1R^2-OR^6]_n + n\ NaHal$$

Das $\alpha$-Hydroxyketon wird in etwa der 4-fachen Gewichtsmenge Dimethylsulfoxid gelöst und unter Kühlung auf 10—20°C und Rühren wird aus zwei Tropftrichtern gleichzeitig 1 Mol-Aequivalent des Alkylhalogenids $R^6Hal$ und 1 Mol-Aequivalent konzentrierte Natronlauge zugetropft. Anschliessend wird 3 Std. bei Raumtemperatur gerührt, dann wird vom ausgeschiedenen Natriumhalogenid abfiltriert, das Filtrat mit Aether verdünnt, mit Wasser gewaschen, über $Na_2SO_n$ getrocknet und eingedampft. Das resultierende Rohprodukt wird durch Säulenchromatographie, Kristallisation oder Vakuumdestillation gereinigt. Beispiele für verwendbare Halogenverbindungen sind Methyljodid, Isopropylbromid, Allylbromid, Cyclohexylbromid, Benzylchlorid oder Chloressigsäureäthylester. Statt eines Alkylhalogenides kann auch ein Dialkylsulfat oder Alkyl-arylsulfonat als Verätherungs-Reagens verwendet werden.

TABELLE 1

| Verbindung | Formel | Herstellungs-methode | Reinigung | Physikalische Eigenschaften |
|---|---|---|---|---|
| 1 | 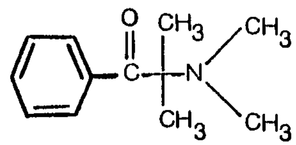 | B+D+G | Dest. | Kp $_{0,2}$ 61° |
| 2 | 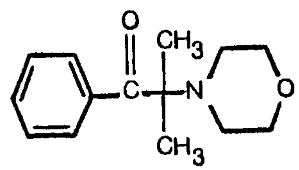 | A+D+G | Krist. (Petr. Aether) | Kp $_{0,02}$ 110–112° Smp. 78–80°C |
| 3 | 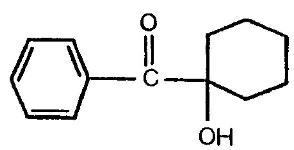 | B+D+E | Krist. (Petr. Aether) | Smp. 42–50° |
| 4 | 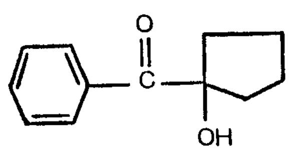 | B+D+E | Chromat. | Oel |

TABELLE 1 (Fortsetzung)

| Verbindung | Formel | Herstellungs-methode | Reinigung | Physikalishe Eigenschaften |
|---|---|---|---|---|
| 5 | 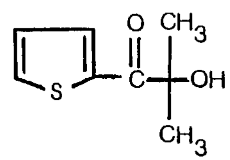 | B+D+E | Dest. | Kp. 10 150° *) |
| 6 | Entfällt | | | |
| 7 | 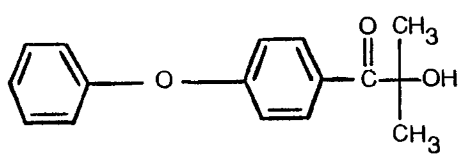 | C+D+E | Dest. | Kp. 0,05 180° *) |
| 8 | 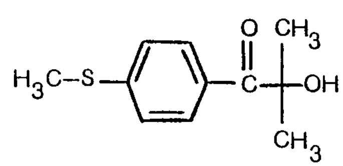 | B+D+E | Dest. | Wachs Kp. 0,1 150° *) |

TABELLE 1 (Fortzetsung)

| Verbindung | Formel | Herstellungs-methode | Reinigung | Physikalische Eigenschaften |
|---|---|---|---|---|
| 9 | 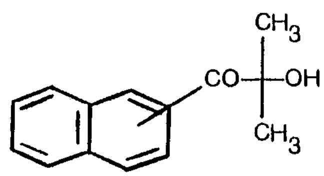 | B+D+E | Dest. | Kp. $_{0,2}$ 140° *)<br>$\alpha$: $\beta$-Isomer 6:1 |
| 10 | 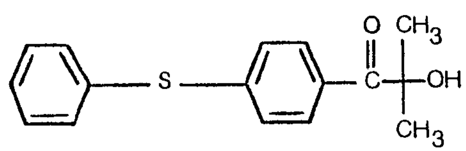 | B+D+E | Dest. | Kp $_{0,1}$ 180° *) |
| 11 | 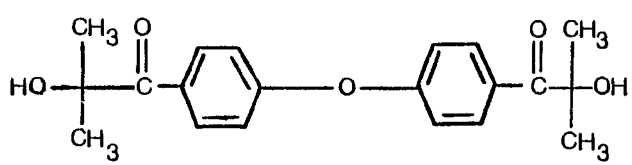 | B+D+E | Dest. | Kp $_{0,05}$ 220° *)<br>Wachs |
| 12 | 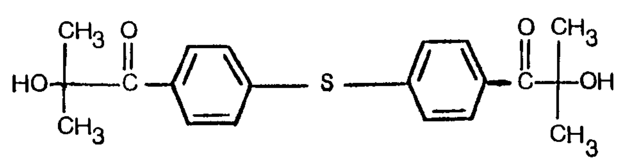 | B+D+E | Krist.<br>(Diäthyläther) | Smp. 90–91° |

TABELLE 1 (Fortzetsung)

| Verbindung | Formel | Herstellungs-methode | Reinigung | Physikalische Eigenschaften |
|---|---|---|---|---|
| 13 | 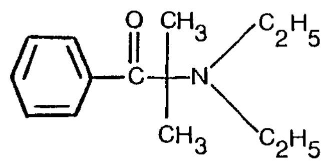 | B+D+G | Dest. | Kp $_{0,1}$ 150° *) |
| 14 | 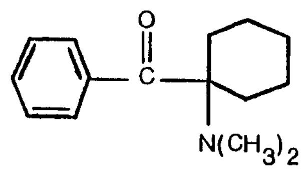 | B+D+G | Chromat. | Oel |
| 15 | 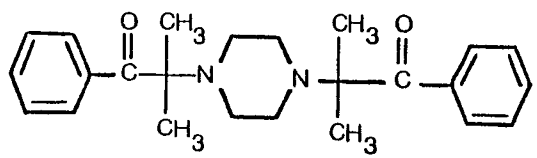 | B+D+G | Krist. (Aethanol) | Smp. 141–143° |
| 16 | 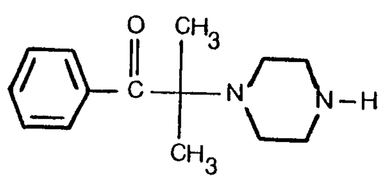 | B+D+G | Dest. | kp $_{0,1}$ 150° *) |

TABELLE 1 (Fortzetsung)

| Verbindung | Formel | Herstellungs-methode | Reinigung | Physikalische Eigenschaften |
|---|---|---|---|---|
| 17 | 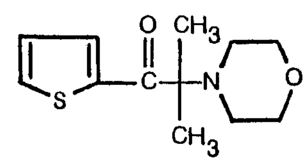 | B+D+G | Dest. | Kp $_{0,1}$ 150° *) |
| 18 | 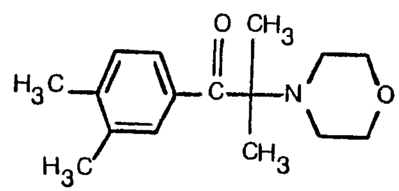 | B+D+G | Krist. (Diisopropyl-äther) | Smp. 110–112° |
| 19 | 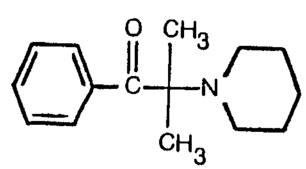 | C+H oder A+D+G | Dest. | Smp. 34° Kp $_{0,05}$ 150° *) |
| 20 | 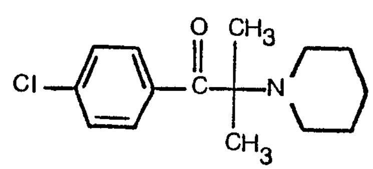 | B+H | Dest. | Kp 0.15 150° *) |

TABELLE 1 (Fortzetsung)

| Verbindung | Formel | Herstellungs-methode | Reinigung | Physikalische Eigenschaften |
|---|---|---|---|---|
| 21 | 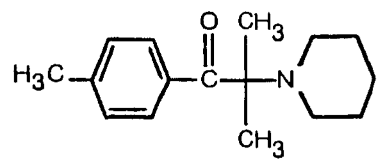 | B+D+G | Dest. | Kp 0,1 150° *) |
| 22 | 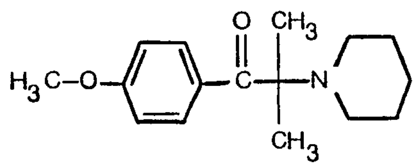 | B+H | Dest. | Kp 0,2 180° *) |
| 23 | 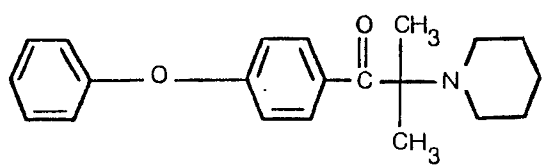 | C+H | Dest. | Kp 0,1 200° *) |
| 24 | 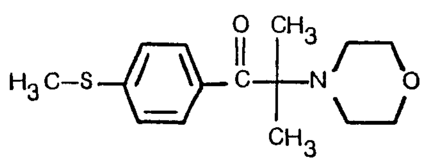 | B+D+G | Dest. | Smp. 68–71° Kp 0,1 210° *) |

TABELLE 1 (Fortzetsung)

| Verbindung | Formel | Herstellungs-methode | Reinigung | Physikalische Eigenschaften |
|---|---|---|---|---|
| 25 | 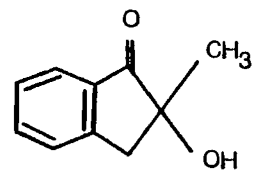 | F | Chromat. | Smp 54° |
| 26 | 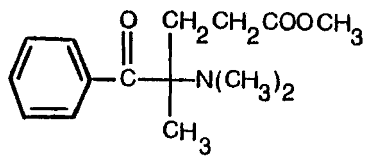 | I | Chromat. | Oel |
| 27 | 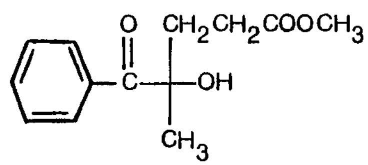 | I | Chromat. | Oel |
| 28 | 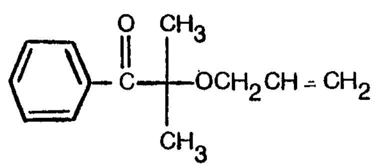 | K | Chromat. | Oel |
| 29 | 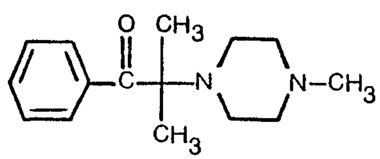 | B+D+G | | Fp 38–41° |

*) Temperatur des Luftbades bei Kugelrohrdestillation.

Beispiel 1

Eine Harzmischung aus 80 Teilen Plex 6616 (Acrylatharz der Firma Röhm, Darmstadt), 20 Teilen Trimethylolpropan-tris-acrylat und 2 Teilen Photoinitiator wird mit einem Filmziehgerät in einer Dicke von 40 $\mu m$ auf Glasplatten aufgezogen. Diese Filme werden ca. 20 Sekunden abgelüftet und anschliessend mit einem Hg-Mitteldruckbrenner (Hanovia-Gerät, Modell 45080) bestrahlt. Dabei werden die Proben auf einem Transportband mit einer solchen Geschwindigkeit unter der UV-Lampe vorbeibewegt, dass sich eine effektive Belichtungszeit von 0,16 Sekunden pro Durchlauf ergibt.

In der folgenden Tabelle 2 sind in der zweiten Spalte die Anzahl Durchläufe (D) angeführt, die notwendig waren, um klebfreie Filme zu erhalten.

In der Dritten Spalte wird die Härte der Filme nach verschiedener Anzahl von Durchläufen, gemessen mit dem Pendelgerät nach König, angegeben.

Die letzte Spalte gibt die Lagerstabilität der Harz-Photoinitiator-Mischung im Dunkeln bei 60°C an.

TABELLE 2

| Verwendeter Photoinitiator | Nötige Durchläufe | Pendelhärte nach König nach Anzahl Durchläufe (D) | | | Lagerstabilität in Tagen |
|---|---|---|---|---|---|
| Nr. 1 | 4 | 78(4D) | 94(6D) | 98(8D) | >30 |
| Nr. 2 | 4 | 101(4D) | 114(4D) | 116(8D) | >30 |
| Nr. 15 | 3 | 95(3D) | 102(4D) | 107(5D) | <30 |
| Nr. 26 | 4 | 47(3D) | 72(4D) | 88(5D) | <30 |
| $\alpha$-Hydroxypropiophenon (Vergleich) | 3 | 68(3D) | 75(4D) | 87(5D) | 1 |
| $\alpha$-Methylbenzoin (Vergleich) | 5 | 49(3D) | 69(4D) | 91(5D) | |
| Benzoin-tert.-butyläther (Vergleich) | 5 | 93(5D) | 106(7D) | 113(9D) | <30 |
| 2-Phenyl-dimethoxyacetophenon (Vergleich) | 6 | 112(6D) | 121(8D) | 130(10D) | >30 |
| p-Methyl-$\alpha$,$\alpha$-dimorpholinoacetophenon (Vergleich) | 8 | 92(8D) | 100(10D) | 109(12D) | <5 |
| $\alpha$,$\alpha$-Dimorpholinoacetophenon (Vergleich) | 17 | 84(17D) | 98(19D) | | <1 |

Beispiel 2

Harzmischungen aus 60 Teilen Uvimer DV—530 (Urethanacrylat der Fa. Polychrom), 37 Teilen Hexandioldiacrylat und 3 Teilen Photoinitiator wurden in einer Filmdicke von 30 $\mu m$ auf Glasplatten aufgezogen und wie in Beispiel 1 beschrieben, belichtet. Dabei wurden folgende Resultate erhalten.

TABELLE 3

| Verwendeter Photoinitiator | Nötige Durchläufe bis zur Wischfertigkeit | Pendelhärte nach König in Abhängigkeit von D |
|---|---|---|
| Nr. 1 | 3 | 129(3D) |
| Nr. 2 | 3 | 157(7D) |
| Diäthoxyacetophenon (Vergleich) | 10 | 156(10D) |
| Benzoin-tert.-butyläther (Vergleich) | 12 | 136(12D) |
| 2-Phenyldimethoxyacetophenon (Vergleich) | 8 | 155(8D) |

Beispiel 3

2% Photoinitiator wurden in einem ungesättigten Polyesterharz (Crystic PKE 306, Fa. Maeder, Killwangen, Schweiz) gelöst. Diese Harzmischungen wurden in einer Filmdicke von 60 $\mu$m auf Glasplatten aufgetragen. Diese Filme wurden, wie in Beispiel 1 beschrieben, belichtet. Die folgende Tabelle 4 gibt die Anzahl Durchläufe durch das Belichtungsgerät bis zur Wischfestigkeit der Filme, sowie die Pendelhärte in Abhängigkeit von D wieder.

TABELLE 4

| Verwendeter Photoinitiator | Nötige Durchläufe bis zur Wischfestigkeit | Pendelhärte nach König in Abhängigkeit von D |
|---|---|---|
| Nr. 1 | 13 | 21(13D) 34(15D) 62(17D) |
| Nr. 2 | 8 | 20(8D) 31(10D) 89(12D) |

Beispiel 4

Eine Harzmischung aus 90 Teilen Laromer LR 8496 (Acrylatharz der Firma BASF, BRD), 10 Teilen Hexandioldiacrylat, 0,5 Teilen ByK 300 (Verlaufshilfsmittel der Firma ByK-Mallinckrodt, BRD) und 3 Teilen Photoinitiator für die Härtung unter Luft bzw. 0,5 Teilen Photoinitiator für die Härtung unter Stickstoff, wird mit einer 15 $\mu$-Spirale elektromotorisch auf Kartons aufgetragen. Nach einer kurzen Abluftzeit wird mit einem UV-Gerät (Modell PPG-QC-Prozessor) mit einer UV-Lampe von 80 Watt/cm ausgehärtet. In der folgenden Tabelle 5 ist die maximale Transportgeschwindigkeit in m/Min angegeben, bei der unter Luft, bzw. unter Stickstoff klebfreie Filme erhalten wurden.

TABELLE 5

| Verwendeter Photoinitiator | Transportgeschwindigkeit (m/Min) Luft | Stickstoff |
|---|---|---|
| Nr. 3 | 20 | 100 |
| Nr. 4 | 20 | 100 |
| Nr. 18 | 10 | 90 |
| Nr. 19 | 20 | 90 |

Beispiel 5

Eine Harzmischung aus 70 Teilen Ebecryl 593 (Polyesteracrylat der Firma UCB, Belgien), 30 Teilen Trimethylolpropantrisacrylat, 0,5 Teilen ByK 300 (Verlaufshilfsmittel der Firma ByK-Mallinckrodt, BRD), und 3 Teilen Photoinitiator wird mit einem Aufziehrahmen in einer Schichtdicke von 30—40 $\mu$ auf Glasplatten aufgetragen. Nach einer kurzen Abluftzeit wird mit einem UV-Laborgerät (Modell PPG/Q.C-Prozessor) mit einer UV-Lampe von 80 Watt/cm ausgehärtet. Nach der UV-Härtung wird 1/2 Stunde im Normklima gelagert und anschliessend die Pendelhärte nach König bestimmt. In der folgenden Tabelle 6 werden die gemessenen Pendelhärten in Abhängigkeit von der Transportgeschwindigkeit unter der Lampe notiert.

TABELLE 6

| Verwendeter Photoinitiator | Pendelhärte in sec. 10 m/Min | 25 m/Min |
|---|---|---|
| Nr. 7 | 160 | 144 |
| Nr. 11 | 155 | 143 |
| Nr. 16 | 162 | 154 |
| Nr. 19 | 129 | 98 |
| Nr. 21 | 146 | 129 |
| Nr. 24 | 134 | 108 |
| Nr. 27 | 139 | 116 |
| Nr. 28 | 153 | 131 |
| Nr. 29 | 164 | 152 |

**Patentansprüche**

1. Verwendung von Verbindungen der Formeln I, II, III oder IV

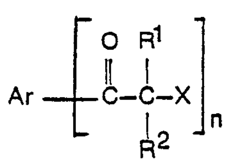
I

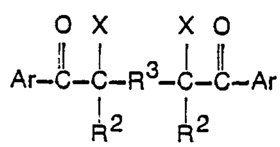
II

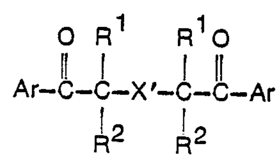
III

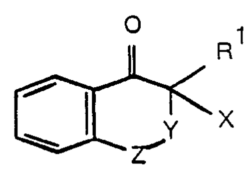
IV

worin n 1 oder 2 ist,

Ar, wenn n = 1 ist, Aryl mit 6—14 C-Atomen, durch Cl, Br, CN, OH, Alk, —OAlK, —SAlk, —$SO_2$Alk, —$SO_2$Phenyl, —COOAlk, —$SO_2NH_2$, —$SO_2$NHAlk, —$SO_2$N(Alk)$_2$, —NHAlk, —N(Alk)$_2$, Phenoxy oder —NHCOAlk substituiertes Phenyl oder Thienyl, Pyridyl oder Furyl darstellt und neue Zeile und einrücken wenn n = 2 ist, Arylen mit 6—12 C-Atomen oder eine Gruppe -Phenylen-T-Phenylen- bedeutet,

Alk einen Alkylrest mit 1—4 C-Atomen bedeutet

X eine der Gruppen —$NR^4R^5$, —$OR^6$, —O—Si($R^7$)($R^8$)$_2$, Hydroxymethoxy ($C_1$—$C_4$-Alkoxy)-methoxy, ($C_2$—$C_8$-Acyloxy)-methoxy oder zusammen mit $R^1$ —OCH($C_1$—$C_8$-Alkyl)—O($CH_2$)$_{1-2}$—, —OCH($C_6$—$C_{14}$-Aryl)—O—($CH_2$)$_{1-2}$—, —OCH($C_1$—$C_8$-Alkyl)— oder —OCH($C_6$—$C_{14}$-Aryl)— darstellt,

X' eine der Gruppen —$NR^{10}$—, —N(Phenyl)—,

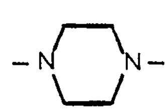

oder —N($R^{10}$)—$R^{11}$—N($R^{10}$)— darstellt,

Y eine direkte Bindung oder —$CH_2$— darstellt,

Z —O—, —S—, —$SO_2$—, —$CH_2$— oder —$C(CH_3)_2$— darstellt,
T —O—, —S—, —$SO_2$—, —$CH_2$— oder —CH=CH— darstellt,
$R^1$ in Formel I bei n gleich 1 und X gleich —$OR^6$ neue Zeile und einrücken $C_1$—$C_8$ Alkyl, das durch $C_2$—$C_8$ Acyloxy, —COO—($C_1$—$C_4$)Alkyl oder —CN substituiert sein kann, $C_5$—$C_6$ Cycloalkyl oder $C_7$—$C_9$ Phenylalkyl bedeutet, neue Zeile und einrücken und in allen anderen Fällen neue Zeile und einrücken Alkyl mit 1—8 C-Atomen, das durch OH, $C_1$—$C_4$ Alkoxy, $C_2$—$C_8$ Acyloxy, —COO—($C_1$—$C_4$)Alkyl oder —CN substituiert sein kann, Cycloalkyl mit 5—6 C-Atomen oder Phenylalkyl mit 7—9 C-Atomen bedeutet,
$R^2$ eine der für $R^1$ gegebenen Bedeutungen hat oder Allyl, Methallyl, 2-Carbamoyläthyl, 2-(N—$C_1$—$C_4$-Alkylcarbamoyl)-äthyl, 2-(N,N—Di—$C_1$—$C_4$-Alkylcarbamoyl)-äthyl, 2-(2-Pyridyl)-äthyl, 2-(2-Oxo-1-pyrrolidinyl)-äthyl oder 2-(Di—O—$C_1$—$C_4$-alkylphosphono)-äthyl ist, neue Zeile $R^1$ und $R_2$ zusammen Alkylen mit 4—6 C-Atomen oder Oxa- oder Azaalkylen mit 3—4 C-Atomen bedeutet, neue Zeile oder $R^1$ und $R^2$ in Formel I beide Hydroxymethyl sind,
$R^3$ eine direkte Bindung, Alkylen mit 1—6 C-Atomen, $C_2$—$C_6$-Oxaalkylen, $C_2$—$C_6$-Thiaalkylen, $C_2$—$C_6$—S-Oxothiaalkylen, $C_2$—$C_6$-S,S-Dioxo-thiaalkylen oder Cyclohexylen bedeutet, neue Zeile oder zusammen mit den beiden Substituenten $R^2$ und den beiden C-Atomen, an die diese Substituenten gebunden sind, einen Cyclopentan- oder Cyclohexanring bildet,
$R^4$ Alkyl mit 1—12 C-Atomen, durch —OH oder —OAlk substituiertes Alkyl mit 2—4 C-Atomen, Allyl, Cyclohexyl, Phenylalkyl mit 7—9 C-Atomen, Phenyl oder durch Cl, Alk, OH, —OAlk oder —COOAlk substituiertes Phenyl bedeutet,
$R^5$ Alkyl mit 1—12 C-Atomen, durch —OH oder —OAlk substituiertes Alkyl mit 2—4 C-Atomen, Allyl, Cyclohexyl oder Phenylalkyl mit 7—9 C-Atomen bedeutet, neue Zeile oder zusammen mit $R^4$ Alkylen mit 4—5 C-Atomen bedeutet, das durch —O—, —NH— oder —NAlk— unterbrochen sein kann, neue Zeile oder im Falle von Verbindungen der Formel I zusammen mit $R^2$ Alkylen oder Phenylalkylen mit 1—9 C-Atomen oder Oxa- oder Azaalkylen mit 2—3 C-Atomen bedeutet,
$R^6$ Wasserstoff, Alkyl mit 1—12 C-Atomen, durch —OH oder —OAlk substituiertes Alkyl mit 2—4 C-Atomen, Allyl, Cyclohexyl, Phenylalkyl mit 7—9 C-Atomen, Phenyl oder durch Cl oder Alk substituiertes Phenyl bedeutet,
$R^7$ und $R^8$ gleich oder verschieden sind und Alkyl mit 1—4 C-Atomen oder Phenyl bedeuten,
$R^{10}$ Alkyl mit 1—8 C-Atomen, Cyclohexyl oder Benzyl und
$R^{11}$ Alkylen mit 2—8 C-Atomen, Xylylen, Phenylen oder eine Gruppe -Phenylen-T-Phenylen- darstellt, neue Zeile mit Ausnahme der Verbindungen der Formel I, worin n = 1 ist und Ar Phenyl oder durch Alkyl, Alkoxy, Dialkylamino, Cl oder Br substituiertes Phenyl ist und X Hydroxy oder Alkoxy ist und $R^1$ und $R^2$ unsubstituiertes Alkyl darstellen,
als Initiatoren für die Photopolymerisation ungesättigter Verbindungen sowie für die photochemische Vernetzung von Polyolefinen.

2. Verwendung gemäss Anspruch 1 wobei in Verbindungen der Formel I oder II, X eine Gruppe —$NR^4R^5$ darstellt.

3. Verwendung gemäss Anspruch 1 wobei in Verbindungen der Formeln I oder II, X eine Gruppe —$OR^6$ ist.

4. Verwendung gemäss Anspruch 1 wobei in Verbindungen der Formel I, II, III oder IV neue Zeile Ar, wenn n = 1 ist, Aryl mit 6 bis 14 C-Atomen oder durch Cl, Br, Alk, —OAlk, —OPhenyl, —COOAlk, —$N(Alk)_2$ oder —NHCOAlk substituiertes Phenyl darstellt und Alk einen Niederalkylrest mit 1—4 C-Atomen bedeutet und neue Zeile und einrücken wenn n = 2 ist, $C_6$—$C_{12}$ Arylen oder eine Gruppe -Phenylen-T-Phenylen- bedeutet, neue Zeile X eine der Gruppen —$NR^4R^5$ oder —$OR^6$ darstellt, neue Zeile X' eine der Gruppen

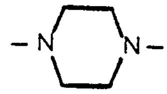

oder —$N(R^{10})$—$R^{11}$—$N(R^{10})$— darstellt, neue Zeile Z —O—, —$CH_2$ oder —$C(CH_3)_2$— darstellt, neue Zeile T —O— oder —$CH_2$— darstellt, neue Zeile $R^1$ in Formel I bei n = 1 und X = —$OR^6$ neue Zeile und einrücken unsubstituiertes oder durch —COOAlk oder CN substituiertes $C_1$—$C_8$ Alkyl, oder $C_7$—$C_9$ Phenylalkyl bedeutet, neue Zeile und einrücken und in allen anderen Fällen neue Zeile und einrücken unsubstituiertes oder durch —OH, OAlk, —COOAlk oder —CN substituiertes $C_1$—$C_8$ Alkyl, oder $C_7$—$C_9$ Phenylalkyl bedeutet, neue Zeile $R^2$ eine der für $R^1$ gegebenen Bedeutungen hat oder Allyl oder eine Gruppe —$CH_2CH_2R^{13}$ ist oder zusammen mit $R^1$ $C_4$—$C_6$ Alkylen oder $C_3$—$C_4$ Oxa- oder Azaalkylen bedeutet, neue Zeile $R^3$ eine direkte Bindung oder $C_1$—$C_6$ Alkylen bedeutet neue Zeile oder zusammen mit den beiden Substituenten $R^2$ und den beiden C-Atomen, an die diese Substituenten gebunden sind, einen Cyclopentan- oder Cyclohexanring bildet, neue Zeile $R^4$ $C_1$—$C_{12}$ Alkyl, durch —OH oder OAlk substituiertes $C_2$—$C_4$ Alkyl, oder Allyl bedeutet, neue Zeile $R^5$ $C_1$—$C_{12}$ Alkyl, durch OH oder OAlk substituiertes $C_2$—$C_4$ Alkyl oder Allyl bedeutet neue Zeile oder zusammen mit $R^4$ $C_4$—$C_5$ Alkylen bedeutet, das durch —O— oder —NAlk— unterbrochen sein kann, neue Zeile $R^6$ Wasserstoff, $C_1$—$C_{12}$ Alkyl, durch OH oder OAlk substituiertes $C_2$—$C_4$ Alkyl, Allyl, Benzyl oder Phenyl bedeutet, neue Zeile $R^{10}$

$C_1$—$C_8$ Alkyl, neue Zeile $R^{11}$ $C_2$—$C_8$ Alkylen und neue Zeile $R^{13}$ —$CONH_2$, —CONHAlk, —$CON(Alk)_2$, —P(O) $(OAlk)_2$ oder 2-Pyridyl bedeuten.

5. Verwendung gemäss Anspruch 4 wobei in Verbindungen der Formeln I, II oder III, neue Zeile Ar, wenn n = 1 ist, $C_6$—$C_{14}$-Aryl oder durch Cl, Br, Alk, oder —OAlk substituiertes Phenyl darstellt und Alk einen Niederalkylrest mit 1—4 C-Atomen bedeutet und neue Zeile und einrücken wenn n = 2 ist, $C_6$—$C_{12}$ Arylen, oder eine Gruppe -Phenylen-T-Phenylen- bedeutet, neue Zeile X' die Gruppe

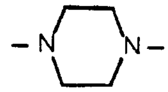

darstellt, neue Zeile T —O— oder —$CH_2$— darstellt, neue Zeile $R^1$ $C_1$—$C_8$ Alkyl bedeutet, neue Zeile $R^2$ $C_1$—$C_8$ Alkyl oder Allyl ist, neue Zeile $R^3$ eine direkte Bindung oder $C_1$—$C_6$ Alkylen ist, neue Zeile $R^4$ $C_1$—$C_{12}$ Alkyl bedeutet, neue Zeile $R^5$ $C_1$—$C_{12}$ Alkyl bedeutet neue Zeile oder zusammen mit $R^4$ $C_4$—$C_5$ Alkylen bedeutet, das durch —O— oder —NAlk— unterbrochen sein kann und neue Zeile $R^6$ Wasserstoff, $C_1$—$C_{12}$ Alkyl, durch OH oder OAlk substituiertes $C_2$—$C_4$ Alkyl, Allyl, Benzyl oder Phenyl bedeutet.

6. Verwendung gemäss Anspruch 1 wobei in Verbindungen der Formel I, worin n = 1 und X = OH ist $R^1$ und $R^2$ zusammen Alkylen mit 2—5 C-Atomen darstellen.

7. Verwendung gemäss Anspruch 1 wobei in Verbindungen der Formel I, n = 2 ist.

8. Verwendung gemäss Anspruch 1 von 2-Hydroxy-2-methyl-(p-phenoxypropiophenon).

9. Verwendung gemäss Anspruch 1 von 1-Benzoyl-cyclohexanol.

10. Verwendung gemäss Anspruch 1 von 4,4'-Bis-($\alpha$-hydroxyisobutyryl)-diphenyloxid.

11. Verwendung gemäss Anspruch 1 von 4,4'-Bis-($\alpha$-hydroxyisobutyryl)-diphenylmethan.

12. Photopolymerisierbares System, bestehend aus mindestens einer ungesättigten photopolymerisierbaren Verbindung und 0,1 bis 20 Gew.-%, vorzugsweise 0,5 bis 5 Gew.-% einer der Verbindungen der Formeln I, II, III oder IV des Anspruches 1 als Photoinitiator sowie gegebenenfalls weiteren bekannten und üblichen Zusatzstoffen.

13. Photopolymerisierbares System gemäss Anspruch 12, dadurch gekennzeichnet, dass es als ungesättigte Verbindung einen oder mehrere Ester der Acryl- oder Methacrylsäure enthält.

14. Photopolymerisierbares System gemäss Anspruch 12, dadurch gekennzeichnet, dass es sich um eine Druckfarbe handelt.

15. Verbindungen der Formel I des Anspruchs 1, worin n = 1 ist, Ar $C_{10}$—$C_{14}$ Aryl, durch CN, —OH, —OPhenyl, —SAlk, —$SO_2$Alk, —$SO_2$Phenyl, —COOAlk, —$SO_2NH_2$, —$SO_2$NHAlk, —$SO_2N(Alk)_2$, —NHAlk oder —NHCOAlk substituiertes Phenyl, Thienyl oder Pyridyl ist und X OH oder —OAlk ist. —OAlk ist.

16. Verbindungen der Formel I des Anspruchs 1, worin n = 1 ist, X eine Gruppe —$OR^6$ und $R^6$ durch OH oder OAlk substituiertes $C_2$—$C_4$ Alkyl, Allyl, Cyclohexyl, Benzyl, unsubstituiertes oder durch Cl oder Alk substituiertes Phenyl bedeutet.

17. Verbindungen der Formel I des Anspruchs 1, worin n = 1 ist, X eine Gruppe —$OSiR^7(R^8)_2$, $R^7$ $C_2$—$C_4$ Alkyl oder Phenyl ist.

18. Verbindungen der Formel I des Anspruchs 1, worin n = 1 ist, Ar Phenyl, Halogenphenyl oder Diphenylyl ist, X eine Gruppe —$NR^4R^5$ ist, $R^1$ $C_2$—$C_8$ Alkyl, durch OH, OAlk, $C_2$—$C_8$ Acyloxy, —COOAlk oder CN substituiertes $C_1$—$C_8$ Alkyl, $C_5$—$C_6$ Cycloalkyl oder $C_7$—$C_9$ Phenylalkyl ist, $R^2$ eine der für $R^1$ angegebene Bedeutung hat oder Allyl ist oder zusammen mit $R^1$ $C_4$—$C_6$ Alkylen oder $C_3$—$C_4$ Oxa- oder Azaalkylen bedeutet.

19. Verbindungen der Formel I des Anspruchs 1, worin n = 1 ist, Ar durch CN, OH, Alk, OAlk, —OPhenyl, —SAlk, —$SO_2$Alk, —$SO_2$Phenyl, —COOAlk, —$SO_2NH_2$, —$SO_2$NHAlk, —$SO_2N(Alk)_2$, —NHAlk, —$N(Alk)_2$ oder —NHCOAlk substituiertes Phenyl, Naphthyl, Thienyl, Pyridyl, Furyl ist und X eine Gruppe —$NR^4R^5$ darstellt.

20. Verbindungen der Formel I des Anspruchs 1, worin n = 2 ist.

21. 2-Hydroxy-2-methyl-(p-phenoxypropiophenon) als Verbindung des Anspruchs 15.

22. 4,4'-Bis-($\alpha$-hydroxy-isobutyryl)-diphenyloxid als Verbindung des Anspruchs 20.

23. 4,4'-Bis-($\alpha$-hydroxy-isobutyryl)-diphenylmethan als Verbindung des Anspruchs 20.

**Claims**

1. The use of compounds of the formulae I, II, III or IV

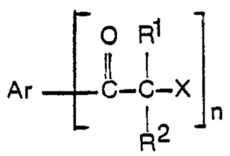

I

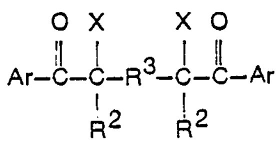

II

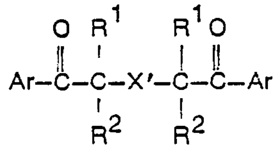

III

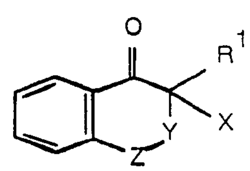

IV

wherein
n is 1 or 2,

Ar if n is 1, represents $C_6$—$C_{14}$ aryl, phenyl which is substituted by Cl, Br, CN, OH, alk, —Oalk, —Salk, —$SO_2$alk, —$SO_2$phenyl, —COOalk, —$SO_2NH_2$, —$SO_2$NHalk, —$SO_2N(alk)_2$, —NHalk, —$N(alk)_2$, phenoxy or —NHCOalk, or represents thienyl, pyridyl or furyl and if n is 2, represents $C_6$—$C_{12}$ arylene or a phenylene-T-phenylene group,

alk represents a $C_1$—$C_4$alkyl radical,

X represents a member selected from the group consisting of —$NR^4R^5$, —$OR^6$, —O—$Si(R^7)(R^8)_2$, hydroxymethoxy, ($C_1$—$C_4$alkoxy)methoxy, ($C_2$—$C_8$acyloxy)methoxy, or X together with $R^1$ is —OCH($C_1$—$C_8$alkyl)—O$(CH_2)_{1-2}$, OCH($C_6$—$C_{14}$aryl)—O—$(CH_2)_{1-2}$, —OCH($C_1$—$C_8$alkyl) or —OCH($C_6$—$C_{14}$aryl),

X′ represents a member selected from the group consisting of —$NR^{10}$, N(phenyl),

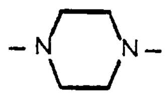

or —$N(R^{10})$—$R^{11}$—$N(R^{10})$,

Y represents a direct bond or —$CH_2$—,

Z represents —O—, —S—, —$SO_2$—, —$CH_2$—, or —$C(CH_3)_2$—, or —CH=CH—,

$R^1$ in formula I, if n is 1 and X is —$OR^6$, represents $C_1$—$C_8$alkyl which is unsubstituted or substituted by $C_2$—$C_8$acyloxy, —COO—$C_1$—$C_4$alkyl or CN, or represents $C_5$—$C_6$cycloalkyl or $C_7$—$C_9$phenylalkyl, and in all other cases represents $C_1$—$C_8$alkyl which can be substituted by —OH, $C_1$—$C_4$alkoxy, $C_2$—$C_8$acylkoxy, —COO—$C_1$—$C_4$alkyl or —CN, or represents $C_5$—$C_6$cycloalkyl or $C_7$—$C_9$phenylalkyl,

$R^2$ has one of the meanings assigned to $R^1$ or is allyl, methyallyl, 2-carbamoylethyl, 2-(N—$C_1$—$C_4$alkylcarbamoyl)ethyl, 2-(N,N—di—$C_1$—$C_4$alkylcarbamoyl)ethyl, 2-(2-pyridyl)ethyl, 2-(2-oxo-1-pyrrolidinyl)ethyl or 2-(di—O—$C_1$—$C_4$alkylphosphono)ethyl; or $R^1$ and $R^2$ together represent $C_4$—$C_6$alkylene or $C_3$—$C_4$oxa- or azaalkylene, or $R^1$ and $R^2$ in formula I are both hydroxymethyl,

$R^3$ represents a direct bond, $C_1$—$C_6$alkylene, $C_2$—$C_6$oxaalkylene, $C_2$—$C_6$thiaalkylene, $C_2$—$C_6$—S-oxothiaalkylene or $C_2$—$C_6$—S,S-dioxothiaalkylene or cyclohexylene, or together with both substituents $R^2$ and both carbon atoms to which these substituents are attached, forms a cyclopentane or cyclohexane ring,

$R^4$ represents $C_1$—$C_{12}$alkyl, $C_2$—$C_4$alkyl substituted by —OH or Oalk, or allyl, cyclohexyl, $C_7$—$C_9$phenylalkyl, phenyl or phenyl which is substituted by Cl, alk, OH, Oalk or —COOalk,

$R^5$ represents $C_1$—$C_{12}$alkyl, $C_2$—$C_4$alkyl which is substituted by —OH or Oalk, or represents allyl, cyclohexyl or $C_7$—$C_9$phenylalkyl, or together with $R^4$ represents $C_4$—$C_5$alkylene which can be interrupted by —O—, —NH— or —Nalk, or, in the case of compounds of the formula I, together with $R^2$ represents $C_1$—$C_9$alkylene or $C_1$—$C_9$phenylalkylene or $C_2$—$C_3$ oxa- or azaalkylene,

$R^6$ represents hydrogen, $C_1$—$C_{12}$alkyl, $C_2$—$C_4$alkyl which is substituted by —OH or —Oalk, or is allyl, cyclohexyl, $C_7$—$C_9$phenylalkyl, phenyl or phenyl substituted by Cl or alk,

$R^7$ and $R^8$ are identical or different and represent $C_1$—$C_4$alkyl or phenyl,

$R^{10}$ represents $C_1$—$C_8$alkyl, cyclohexyl or benzyl,

$R^{11}$ represents $C_2$—$C_8$alkylene, xylylene, phenylene or a -phenylene-T-phenylene group, with the exception of compounds of the formula I, wherein n is 1 and Ar is phenyl or phenyl substituted by alkyl, alkoxy, dialkylamino, Cl or Br, X is hydroxy or alkoxy, and $R^1$ and $R^2$ represent unsubstituted alkyl,

as sensitizers for the photopolymerisation of unsaturated compounds and for the photochemical crosslinking of polyolefins.

2. The use according to claim 1, wherein X is an —$NR^4R^5$ group in compounds of the formula I or II.

3. The use according to claim 1, wherein X is an —$OR^6$ group in compounds of the formula I or II.

4. The use according to claim 1, wherein in compounds of the formula I, II, III or IV,

Ar, if n is 1, represents $C_6$—$C_{14}$aryl, or phenyl which is substituted by Cl, Br, alk, —Oalk, —OPhenyl, —COOalk, —$N(Alk)_2$ or —NHCOalk and alk represents a lower alkyl radical of 1 to 4 carbon atoms, and, if n is 2, represents $C_6$—$C_{12}$arylene or a -phenylene-T-phenylene group,

X represents one of the groups —$NR^4R^5$ or —$OR^6$,

X′ represents one of the groups

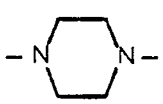

or —$N(R^{10})$—$R^{11}$—$N(R^{10})$—,

Z represents —O—, —$CH_2$— or —$C(CH_3)_2$—,

T represents —O— or —$CH_2$—,

$R^1$ in formula I, if n is 1 and X is —$OR^6$, represents $C_1$—$C_8$alkyl which is unsubstituted or substituted by —COOalk or CN, or represents $C_7$—$C_9$phenylalkyl, and in all other cases represents $C_1$—$C_8$alkyl

which is unsubstituted or substituted by —OH, Oalk, —COOalk or —CN, or is $C_7$—$C_9$phenylalkyl,
$R^2$ has one of the meanings assigned to $R^1$ or is allyl or a —$CH_2CH_2R^{13}$ group, or together with $R^1$ represents $C_4$—$C_6$alkylene or $C_3$—$C_4$oxa- or azaalkylene,
$R^3$ represents a direct bond or $C_1$—$C_6$alkylene, or together with both substituents $R^2$ and both carbon atoms to which these substituents are attached forms a cyclopentane or cyclohexane ring,
$R^4$ represents $C_1$—$C_{12}$alkyl, $C_2$—$C_4$alkyl which is substituted by OH or Oalk or represents allyl,
$R^5$ represents $C_1$—$C_{12}$alkyl, $C_2$—$C_4$alkyl which is substituted by OH or Oalk or represents allyl, or together with $R^4$ represents $C_4$—$C_5$alkylene which can be interrupted by —O— or —Nalk—,
$R^6$ represents hydrogen, $C_1$—$C_{12}$alkyl, $C_2$—$C_4$alkyl which is substituted by OH or Oalk, or is allyl, benzyl, phenyl,
$R^{10}$ represents $C_1$—$C_8$alkyl,
$R^{11}$ represents $C_2$—$C_8$alkylene, and
$R^{13}$ represents —$CONH_2$, —CONHalk, —CON$(alk)_2$, —P(O)$(Oalk)_2$ or 2-pyridyl.

5. The use according to claim 4, wherein in compounds of the formulae I, II or III,
Ar, if n is 1, represents $C_6$—$C_{14}$aryl or phenyl which is unsubstituted or substituted by Cl, Br, alk or —Oalk, and alk represents a lower alkyl radical or 1 to 4 carbon atoms, and, if n is 2, represents $C_6$—$C_{12}$arylene or a -phenylene-T-phenylene group,
X′ represents the group

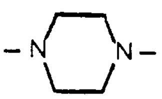 ,

T represents —O— or —$CH_2$—,
$R^1$ represents $C_1$—$C_8$alkyl,
$R^2$ represents —$C_1$—$C_8$alkyl or allyl,
$R^3$ represents a direct bond or $C_1$—$C_6$alkylene,
$R^4$ represents $C_1$—$C_{12}$alkyl,
$R^5$ represents $C_1$—$C_{12}$alkyl or together with R represents $C_4$—$C_5$alkylene which can be interrupted by —O— or —Nalk—, and
$R^6$ represents hydrogen, $C_1$—$C_{12}$alkyl, $C_2$—$C_4$alkyl which is substituted by OH or Oalk, or represents allyl, benzyl or phenyl.

6. The use according to claim 1, wherein in compounds of the formula I, wherein n is 1 and X is OH, $R^1$ and $R^2$ together represent alkylene of 2 to 5 carbon atoms.

7. The use according to claim 1, wherein in compounds of the formula I n is 2.

8. The use according to claim 1 of 2-hydroxy-2-methyl-(p-phenoxypropiophenone).

9. The use according to claim 1 of 1-benzoylcyclohexanol.

10. The use according to claim 1 of 4,4′-bis-($\alpha$-hydroxyisobutyryl)diphenyl oxide.

11. The use according to claim 1 of 4,4′-bis-($\alpha$-hydroxyisobutyryl)diphenylmethane.

12. A photopolymerisable system consisting of at least one unsaturated photopolymerisable compound and 0.1 to 20% by weight, preferably 0.5 to 5% by weight, of a compound of the formula I, II, III or IV according to claim 1 as photosensitizer, and if desired, further known and conventional ingredients.

13. A photopolymerisable system according to claim 12, which contains one or more esters of acrylic or methacrylic acid as unsaturated compound.

14. A photopolymerisable system according to claim 12, which is a printing ink.

15. A compound of the formula I according to claim 1, wherein n is 1, Ar represents $C_{10}$—$C_{14}$aryl, phenyl which is substituted by CN, —OH, Ophenyl, —Salk, —$SO_2$alk, —$SO_2$phenyl, —COOalk, —$SO_2NH_2$, —$SO_2$NHalk, —$SO_2$N$(alk)_2$, —NHalk, or —NHCOalk or represents thienyl or pyridyl, and X is OH or —Oalk.

16. A compound of the formula I according to claim 1, wherein n is 1, X is a —$OR^6$ group and $R^6$ represents $C_2$—$C_4$alkyl which is substituted by OH or Oalk or represents allyl, cyclohexyl, benzyl, phenyl which is unsubstituted or substituted by Cl or alk.

17. A compound of the formula I according to claim 1, wherein n is 1, X is a —$OSiR^7$ $(R^8)_2$ group, and $R^7$ is $C_2$—$C_4$ alkyl or phenyl.

18. A compound of the formula according to claim 1, wherein n is 1, Ar is phenyl, halophenyl or diphenylyl, X is a —$NR^4R^5$ group, $R^1$ is $C_2$—$C_8$alkyl, $C_1$—$C_8$alkyl which is substituted by OH, Oalk, $C_2$—$C_8$acyloxy, —COOalk or CN, or is $C_5$—$C_6$cycloalkyl or $C_7$—$C_9$phenylalkyl, $R^2$ has one of the meanings assigned to $R^1$ or is allyl or together with $R^1$ is $C_4$—$C_6$alkylene or $C_3$—$C_4$oxa- or azaalkylene.

19. A compound of the formula I according to claim 1, wherein n is 1, Ar is phenyl which is substituted by CN, OH, alk, Oalk, Ophenyl, —Salk, —$SO_2$alk, —$SO_2$phenyl, —COOalk, —$SO_2NH_2$, —$SO_2$NHalk, —$SO_2$N$(alk)_2$, —NHalk, —N$(alk)_2$ or —NHCOalk, or is naphthyl, thienyl, pyridyl or furyl, and X is a —$NR^4R^5$ group.

20. A compound of the formula I according to claim 1, wherein n is 2.

21. 2-Hydroxy-2-methyl-(p-phenoxypropiophenone) as a compound of claim 15.

22. 4,4′-Bis-($\alpha$-hydroxy-isobutyryl)diphenyl oxide as a compound of claim 20.

23. 4,4′-Bis-($\alpha$-hydroxy-isobutyryl)-diphenyl methane as a compound of claim 20.

**Revendications**

1. Application de composés répondant à l'une des formules I, II, III et IV:

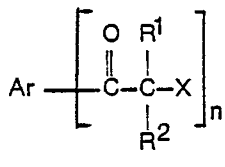 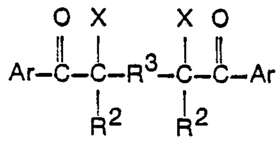 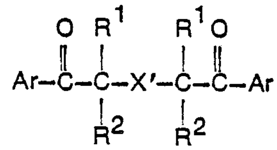 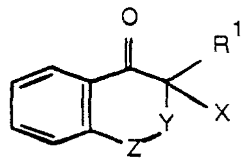

I II III IV

dans lesquelles

n est égal à 1 ou à 2,

Ar représente, dans le cas où n est égal à 1, un aryle en $C_6$—$C_{14}$, un radical phényle ou thiényle éventuellement porteur d'un atome de chlore ou de brome ou d'un radical —CN, —OH, —Alk, —OAlk, —SAlk, —$SO_2$Alk, —$SO_2$phényl, —COOAlk, —$SO_2NH_2$, —$SO_2$NHAlk, —$SO_2N(Alk)_2$, —NHAlk, —$N(Alk)_2$, phénoxy ou —NHCOAlk, ou un radical pyridyle ou furyle, et, dans le cas où n est ègal à 2, un arylène en $C_6$—$C_{12}$ ou un radical -phénylène-T-phénylène-,

Alk représente un radical alkyle contenant de 1 à 4 atomes de carbone,

X représente un radical —$NR^4R^5$, —$OR^6$, —O—$Si(R^7)(R^8)_2$, hydroxyméthoxy, ($C_1$—$C_4$-alcoxy)-méthoxy ou ($C_2$—$C_8$-acyloxy)-méthoxy, ou forme avec $R_1$ un radical —OCH($C_1$—$C_8$-alkyl)—O$(CH_2)_{1-2}$—, —OCH($C_6$—$C_{14}$-aryl)—O—$(CH_2)_{1-2}$—, —OCH($C_1$—$C_8$-alkyl)— ou —OCH($C_6$—$C_{14}$-aryl)—

X' représente un radical —$NR^{10}$—, —N(phényl)—,

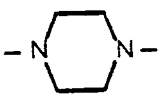

ou —$N(R^{10})$—$R^{11}$—$N(R^{10})$—,

Y représente une liaison directe ou —$CH_2$—,

Z représente —O—, —S—, —$SO_2$—, —$CH_2$— ou —$C(CH_3)_2$—,

T représente —O—, —S—, —$SO_2$—, —$CH_2$— ou —CH=CH—,

$R^1$, dans la formule I, lorsque n est égal à 1 et que X désigne un radical —$OR^6$, représente un alkyle en $C_1$—$C_8$ éventuellement porteur d'un acyloxy en $C_2$—$C_8$, d'un alcoxycarbonyle à alkyle en $C_1$—$C_4$ ou d'un radical cyano, un cycloalkyle en $C_5$ ou $C_6$ ou un phènylalkyle en $C_7$—$C_9$, et dans tous les autres cas un alkyle en $C_1$—$C_8$ éventuellement porteur d'un OH, d'un alcoxy en $C_1$—$C_4$, d'un acyloxy en $C_2$—$C_8$, d'un alcoxycarbonyle à alkyle en $C_1$—$C_4$ ou d'un —CN, un cycloalkyle en $C_5$ ou $C_6$ ou un phénylalkyle en $C_7$—$C_9$,

$R^2$ a l'une des significations qui ont été données ci-dessus pour $R^1$ ou représente un radical allyle, méthallyle, carbamoyl-2 éthyle, (N—$C_1$—$C_4$-alkylcarbamoyl)-2 éthyle, (N,N—di—$C_1$—$C_4$-alkylcarbamoyl)-2 éthyle, (pyridyl-2)-2 éthyle, (oxo-2 pyrrolidinyl-1)-2 éthyle ou (di—O—$C_1$—$C_4$-alkylphosphono)-2 éthyle, ou $R^1$ et $R^2$ forment ensemble un alkylène en $C_4$—$C_6$ ou un oxa- ou aza-alkylène en $C_3$ ou $C_4$, ou $R^1$ et $R^2$ dans la formule I représentent chacun en même temps un radical hydroxyméthyle,

$R^3$ représente une liaison directe, un alkylène en $C_1$—$C_6$, un oxa-alkylène en $C_2$—$C_6$, un thia-alkylène en $C_2$—$C_6$, un S-oxo-thia-alkylène en $C_2$—$C_6$, un S,S-dioxo-thia-alkylène en $C_2$—$C_6$ ou un cyclohexylène, ou forme, avec les deux substituants $R^2$ et les deux atomes de carbone porteurs de ces substituants, un noyau de cyclopentane ou de cyclohexane,

$R^4$ représente un alkyle en $C_1$—$C_{12}$, un alkyle en $C_2$—$C_4$ porteur d'un hydroxy ou d'un —OAlk, un allyle, un cyclohexyle, un phényl-alkyle en $C_7$—$C_9$, un phényle ou un phényle porteur d'un atome de chlore ou d'un radical —Alk, —OH, —OAlk ou —COOAlk,

$R^5$ représente un alkyle en $C_1$—$C_{12}$, un alkyle en $C_2$—$C_4$ porteur d'un radical hydroxy ou —OAlk, un allyle, un cyclohexyle ou un phénylalkyle en $C_7$—$C_9$, ou forme avec $R^4$, un alkylène en $C_4$ ou $C_5$ qui peut être interrompu par —O—, —NH— ou —NAlk—, ou, dans le cas de composés de formule I, forme, avec $R^2$, un alkylène ou un phénylalkylène en $C_1$—$C_9$ ou un oxa- ou aza-alkylène en $C_2$ ou $C_3$,

$R^6$ représente l'hydrogène, un alkyle en $C_1$—$C_{12}$, un alkyle en $C_2$—$C_4$ porteur d'un radical hydroxy ou —OAlk, un allyle, un cyclohexyle, un phénylalkyle en $C_7$—$C_9$, un phényle ou un phényle porteur de Cl ou Alk,

$R^7$ et $R^8$ représentent chacun, indépendamment l'un de l'autre, un alkyle en $C_1$—$C_4$ ou un phényle,

$R^{10}$ représente un alkyle en $C_1$—$C_8$, un cyclohexyle ou un benzyle et

$R^{11}$ représente un alkylène en $C_2$—$C_8$, un xylylène, un phénylène ou un radical -phénylène-T-phénylène, à l'exception des composés de formule I dans lesquels n est égal à 1, Ar représente un radical phényle éventuellement porteur d'un alkyle, d'un alcoxy, d'un dialkylamino ou d'un atome de chlore ou de brome, X représente un hydroxy ou un alcoxy, et $R^1$ et $R^2$ représentent chacun un alkyle non substitué,

en tant qu'amorceurs pour la photopolymérisation de composés insaturés ainsi que pour la réticulation photochimique de polyoléfines.

2. Application selon la revendication 1, caractérisée en ce que, dans des composés de formule I ou II, X représente un radical $—NR^4R^5$.

3. Application selon la revendication 1, caractérisée en ce que, dans des composés de formule I ou II, X représente un radical $—OR^6$.

4. Application selon la revendication 1, caractérisée en ce que, dans des composés de formule I, II, III ou IV:

Ar représente, dans le cas où n est égal à 1, un aryle contenant de 6 à 14 atomes de carbone ou un phényle porteur d'un atome de chlore ou de brome ou d'un radical Alk, —OAlk, —OPhényl, —COOAlk, $—N(Alk)_2$ ou —NHCOAlk, "Alk" désignant un radical alkyle inférieur contenant de 1 à 4 atomes de carbone, et, dans le cas où n est égal à 2, Ar représente un arylène en $C_6—C_{12}$ ou un radical -phénylène-T-phénylène-,

X représente un radical $—NR^4R^5$ ou $—OR^6$,

X' représente un radical

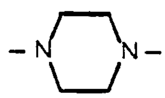

ou $—N(R^{10})—R^{11}—N(R^{10})—$

Z représente —O—, $—CH_2—$ ou $—C(CH_3)_2—$,

T représente —O— ou $—CH_2—$,

$R^1$ représente, dans la formule I, dans le cas où X représente $—OR^6$ et où n est égal à 1, un alkyle en $C_1—C_8$ non substitué ou porteur d'un radical —COOAlk ou —CN, ou un phénylalkyle en $C_7—C_9$, et, dans tous les autres cas, un alkyle en $C_1—C_8$ non substitué ou porteur d'un radical —OH, —OAlk, —COOAlk ou —CN, ou un phénylalkyle en $C_7—C_9$,

$R^2$ a l'une des significations indiquées ci-dessus pour $R^1$ ou représente un allyle ou un radical $—CH_2CH_2R^{13}$, ou encore forme, avec $R^1$, un alkylène en $C_4—C_6$ ou un oxa- ou aza-alkylène en $C_3$ ou $C_4$,

$R^3$ représente une liaison directe ou un alkylène en $C_1—C_6$, ou forme, avec les deux substituants $R^2$ et les deux atomes de carbone auxquels ces substituants sont liés, un noyau de cyclopentane ou de cyclohexane,

$R^4$ représente un alkyle en $C_1—C_{12}$, un alkyle en $C_2—C_4$ porteur d'un radical —OH ou —OAlk, ou un allyle,

$R^5$ représente un alkyle en $C_1—C_{12}$, un alkyle en $C_2—C_4$ porteur d'un radical —OH ou —OAlk, ou un allyle, ou forme, avec $R^4$, un alkylène en $C_4$ ou $C_5$ qui peut être interrompu par —O— ou —NAlk—,

$R^6$ représente l'hydrogène, un alkyle en $C_1—C_{12}$, un alkyle en $C_2—C_4$ porteur d'un radical —OH ou —OAlk, un allyle, un benzyle ou un phényle,

$R^{10}$ représente un alkyle en $C_1—C_8$,

$R^{11}$ représente un alkylène en $C_2—C_8$ et

$R^{13}$ représente un radical $—CONH_2$, —CONHAlk, $—CON(Alk)_2$, $—P(O)(OAlk)_2$ ou pyridyle-2.

5. Application selon la revendication 4, caractérisée en ce que, dans des composés de formule I, II, ou III:

Ar représente, dans le cas où n est égal à 1, un aryle en $C_6—C_{14}$ ou un phényle porteur d'un atome de chlore ou de brome ou d'un radical Alk ou —OAlk ("Alk" désignant un radical alkyle inférieur contenant de 1 à 4 atomes de carbone), et, dans le cas où n est égal à 2, un arylène en $C_6—C_{12}$ ou un radical -phénylène-T-phénylène-,

X' représente le radical

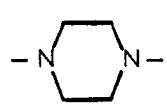 ,

T représente —O— ou $—CH_2—$,

$R^1$ représente un alkyle en $C_1—C_8$,

$R^2$ représente un alkyle en $C_1—C_8$ ou un allyle,

$R^3$ représente une liaison directe ou un alkylène en $C_1—C_6$,

$R^4$ représente un alkyle en $C_1—C_{12}$,

$R^5$ représente un alkyle en $C_1—C_{12}$ ou forme, avec $R^4$, un alkylène en $C_4$ ou $C_5$ éventuellement interrompu par —O— ou —NAlk—, et

$R^6$ représente l'hydrogène, un alkyle en $C_1—C_{12}$, un alkyle en $C_2—C_4$ porteur d'un radical —OH ou —OAlk, un allyle, un benzyle ou un phényle.

6. Application selon la revendication 1, caractérisée en ce que, dans des composés de formule I pour lesquels n est égal à 1 et X représente un radical —OH, $R^1$ et $R^2$ forment ensemble un alkylène contenant de 2 à 5 atomes de carbone.

7. Application selon la revendication 1, caractérisée en ce que, dans des composés de formule I, n est égal à 2.

8. Application selon la revendication 1 de l'hydroxy-2 méthyl-2 (p-phénoxypropiophénone).

9. Application selon la revendication 1 du benzoyl-1 cyclohexanol.

10. Application selon la revendication 1 de l'oxyde de bis-[($\alpha$-hydroxy-isobutyryl)-4 phényle].

11. Application selon la revendication 1 du bis-($\alpha$-hydroxy-isobutyryl)-4,4'-diphénylméthane.

12. Système photopolymérisable constitué d'au moins un composé insaturé photopolymérisable et de 0,1 à 20% en poids, de préférence de 0,5 à 5% en poids, d'un des composés de formule I, II, III ou IV définis à la revendication 1, en tant que photo-amorceur, et éventuellement aussi d'autres additifs connus et usuels.

13. Système photopolymérisable selon la revendication 12, caractérisé en ce qu'il contient, comme composé insaturé, un ou plusieurs esters de l'acide acrylique ou de l'acide méthacrylique.

14. Système photopolymérisable selon la revendication 12, caractérisé en ce qu'il s'agit d'une encre d'imprimerie.

15. Composés de formule I selon la revendication 1, dans lesquels n est égal à 1, Ar représente un aryle en $C_{10}$—$C_{14}$, un phényle porteur d'un radical —CN, —OH, —OPhényl, —SAlk, —$SO_2$Alk, —$SO_2$Phényl, —COOAlk, —$SO_2NH_2$, —$SO_2$NHAlk, —$SO_2N(Alk)_2$, —NHAlk ou NHCOAlk, un thiényle ou un pyridyle, et X représente un radical —OH ou —OAlk.

16. Composés de formule I selon la revendication 1, dans lesquels n est égal à 1, X représente un radical —$OR^6$ dans lequel $R^6$ représente un radical alkyle en $C_2$—$C_4$ porteur d'un —OH ou d'une —OAlk, un allyle, un cyclohexyle, un benzyle, un phényle non substitué ou un phényle porteur d'un atome de chlore ou d'un Alk.

17. Composés de formule I selon la revendication 1, dans lesquels n est égal à 1 et X représente un radical —$OSiR^7(R^8)_2$ dans lequel $R^7$ désigne un alkyle en $C_2$—$C_4$ ou un phényle.

18. Composés de formule I selon la revendication 1, dans lesquels n est égal à 1, Ar représente un radical phényle, halogénophényle ou biphénylyle, X représente un radical —$NR^4R^5$, $R^1$ représente un alkyle en $C_2$—$C_8$, un alkyle en $C_1$—$C_8$ porteur d'un —OH, d'un —OAlk, d'un acyloxy en $C_2$—$C_8$, d'un —COOAlk ou d'un —CN, un cycloalkyle en $C_5$ ou $C_6$ ou un phénylalkyle en $C_7$—$C_9$, et $R^2$ a l'une des significations indiquées pour $R^1$ ou représente un allyle ou encore forme avec $R^1$ un alkylène en $C_4$—$C_6$ ou un oxa- ou aza-alkylène en $C_3$ ou $C_4$.

19. Composés de formule I selon la revendication 1, dans lesquels n est égal à 1, Ar représente un phényle porteur d'un radical —CN, —OH, —Alk, —OAlk, —OPhényl, —SAlk, —$SO_2$Alk, —$SO_2$Phényl, —COOAlk, —$SO_2NH_2$, —$SO_2$NHAlk, —$SO_2N(Alk)_2$, —NHAlk, $N(Alk)_2$ ou —NHCOAlk, un naphtyle, un thiényle, un pyridyle ou un furyle, et X représente un radical —$NR^4R^5$.

20. Composés de formule I selon la revendication 1, dans lesquels n est égal à 2.

21. Composé selon la revendication 15, en l'espèce l'hydroxy-2 méthyl-2 (p-phénoxypropiophénone).

22. Composé selon la revendication 20, en l'espèce l'oxyde de bis-[($\alpha$-hydroxy-isobutyryl)-4 phényle].

23. Composé selon la revendication 20, en l'espèce le bis-($\alpha$-hydroxy-isobutyryl)-4,4' diphényl-méthane.